# EUROPEAN PATENT APPLICATION

(11) **EP 2 850 992 A1**
(43) Date of publication of application: **25.03.2015**
(21) Application number: 13791402.4
(22) Date of filing: 13.05.2013
(51) Int. Cl.: A61B 1/00

(54) **CAPSULE THERAPY DEVICE AND THERAPY SYSTEM**

(30) Priority: 14.05.2012 JP 2012110788
(71) Applicant: Olympus Medical Systems Corp., Tokyo 151-0072 (JP)
(72) Inventor: IKAI, Takuto, Tokyo 151-0072 (JP); KAWANO, Hironao, Tokyo 151-0072 (JP); CHIBA, Atsushi, Tokyo 151-0072 (JP); KIMURA, Atsushi, Tokyo 151-0072 (JP)
(74) Representative: Gunzelmann, Rainer
(86) International application number: PCT/JP2013/063330
(87) International publication number: WO 2013/172312

(57) **Abstract**

Provided is a capsule medical device and a medical system that can alleviate electric power consumed for generating a magnetic field for detecting a position and a posture of the capsule medical device from the outside of a subject. The capsule medical device is a capsule endoscope 100 introduced into a subject and used therein and includes: a power supply unit 123; a capsule detection magnetic field generation unit 126 generating a capsule detection magnetic field for detecting a position and/or a posture of the capsule endoscope 100 from the outside of the subject upon receiving electric power provided by the power supply unit 123; a guiding-magnetic field detector 125 detecting a state of the capsule endoscope 100; and a capsule detection magnetic field controller 127 controlling the power supply to the capsule detection magnetic field generation unit 126 based on an output result of the guiding-magnetic field detector 125.

## Description

### Field

The present invention relates to a capsule medical device and a medical system including a capsule medical device introduced into a subject and acquiring information about the inside of the subject.

### Background

In the past, in the field of endoscopes, a capsule medical device (a capsule endoscope) formed to be of such size that can be introduced into a digestive tract of a subject such as a patient has been developed. The capsule medical device has an image-capturing function and a wireless communication function inside of a capsule-shaped casing, and after the capsule medical device is swallowed from the mouth of the subject, the capsule medical device moves within the digestive tract due to peristaltic movement and the like, during which the capsule medical device successively acquires image data of images inside of the internal organs of the subject (which may be hereinafter also referred to as in-vivo images), and wirelessly transmits the image data to a receiving device that is provided outside of the subject (see, for example, Patent Literature 1). The image data received by the receiving device are retrieved into an image display apparatus and are subjected to predetermined image processing. Accordingly, the in-vivo images are displayed on a display as a still picture display or a moving picture display. A user such as a doctor or a nurse observes an in-vivo image displayed on the image display apparatus as described above to diagnose the state of the internal organs of the subject.

In recent years, a guidance system for guiding a capsule endoscope introduced into a subject using magnetic force (which will be hereinafter referred to as magnetic guidance) has been suggested (see, for example, Patent Literature 2). In general, in such guidance system, a permanent magnet is provided inside of the capsule endoscope, and a guidance device that has a magnetic field generation unit such as an electromagnet is provided outside of the subject. The magnetic field that is generated by the magnetic field generation unit is applied to the permanent magnet provided inside of the capsule endoscope, and the capsule endoscope is magnetically guided to a desired position by magnetic attraction generated from the magnetic field.

For example, the position and the posture of the capsule endoscope in the subject can be detected as follows: a magnetic field generation coil is provided inside of the capsule endoscope, and the position and the posture of the capsule endoscope in the subject can be detected by detecting, outside of the subject, the magnetic field generated by providing an electric power to this coil.

### Citation List

### Patent Literature

Patent Literature 1: Japanese Laid-open Patent Publication No. 2006-75536
Patent Literature 2: Japanese Laid-open Patent Publication No. 2008-119253

### Summary

### Technical Problem

Normally, the capsule medical device is operated by a battery equipped inside a casing. Power supplied from the battery operates the above-described magnetic field generation coil, an imaging element imaging a subject and an illumination element illuminating inside the subject. In addition to that, the power is consumed for a main operation such as wirelessly transmitting image data acquired by the imaging. Therefore, it is desired to alleviate poser consumption of the magnetic field generation coil and to efficiently use the power that can be supplied by the battery.

The present invention has been made considering the above and an object thereof is to provide a capsule medical device and a medical system that can alleviate electric power consumed for generating a magnetic field for detecting a position and a posture of the capsule medical device from the outside of a subject.

### Solution to Problem

To solve the above-described problem and achieve the object, a capsule medical device according to the present invention is introduced into a subject and used therein, and includes: a power supply unit; a capsule detection magnetic field generation unit configured to generate a capsule detection magnetic field, with which a position and/or a posture of the capsule medical device is detected outside the subject, upon receiving electric power provided by the power supply unit; a state detection unit configured to detect a state of the capsule medical device; and a control unit configured to control power supply from the power supply unit to the capsule detection magnetic field generation unit based on a detection result of the state detection unit.

The above-described capsule medical device further includes a magnetic field response unit made of a magnetic member and configured to respond to a guiding-magnetic field which is a magnetic field applied from outside to the capsule medical device for guiding at least one of the position and the posture of the capsule medical device. The state detection unit includes a guiding-magnetic field detector configured to detect at least one of a strength, a direction, and a gradient of the guiding-magnetic field, and the control unit controls the power supply in accordance with a detection result of the guiding-magnetic field detector.

In the above-described capsule medical device, the guiding-magnetic field detector detects the strength of the guiding-magnetic field, and the control unit controls the power supply provided to the capsule detection magnetic field generation unit in accordance with the strength.

In the above-described capsule medical device, the guiding-magnetic field detector detects change in the strength of the guiding-magnetic field, and the control unit controls the power supply provided to the capsule detection magnetic field generation unit in accordance with an amount of the change of the strength.

In the above-described capsule medical device, the guiding-magnetic field detector detects gradient of the guiding-magnetic field, and the control unit controls the power supply provided to the capsule detection magnetic field generation unit in accordance with the gradient.

In the above-described capsule medical device, the guiding-magnetic field detector detects a direction of the guiding-magnetic field, and the control unit controls the power supply provided to the capsule detection magnetic field generation unit in accordance with a magnitude of an angle formed by the direction and a magnetization direction of the magnetic field response unit.

In the above-described capsule medical device, the state detection unit includes a motion detector configured to detect motion of the capsule medical device, and The control unit controls the power supply based on the motion of the capsule medical device detected by the motion detector.

In the above-described capsule medical device, the motion detector is configured to detect a physical quantity corresponding to acceleration of the capsule medical device, and the control unit is configured to reduce turns off the power supply provided to the capsule detection magnetic field generation unit when the acceleration determined from a determination result of the motion detector is either equal to or more than a predetermined value or equal to or less than the predetermined value.

The above-described capsule medical device further includes a magnetic field response unit made of a magnetic member and configured to respond to a guiding-magnetic field which is a magnetic field applied from outside to the capsule medical device for guiding the capsule medical device, and the state detection unit includes: a guiding-magnetic field detector configured to detect the guiding-magnetic field; a motion detector configured to detect motion of the capsule medical device; and a comparison determination unit configured to compare a guiding-magnetic field detection result of the guiding-magnetic field detector and a motion detection result of the motion detector. The control unit controls the power supply based on a result acquired by comparing the guiding-magnetic field detection result of the guiding-magnetic field detector and the motion detection result of the motion detector.

In the above-described capsule medical device, the control unit decreases the power supply provided to the capsule detection magnetic field generation unit when the direction of the gradient of the guiding-magnetic field and the moving direction of the capsule medical device are determined to be the same.

In the above-described capsule medical device, the control unit decreases the power supply provided to the capsule detection magnetic field generation unit when the direction of the guiding-magnetic field and the posture of the capsule medical device are determined to be the same.

In the above-described capsule medical device, the state detection unit includes a distance acquiring unit configured to acquire a distance between the capsule medical device and the subject, and the control unit controls the power supply based on the distance acquired by the distance acquiring unit.

In the above-described capsule medical device, the control unit decreases the power supply provided to the capsule detection magnetic field generation unit when the distance is either equal to or more than a predetermined value or equal to or less than the predetermined value.

In the above-described capsule medical device, the state detection unit includes an internal organ determination unit configured to determine a type of an internal organ of the subject where the capsule medical device passes, and the control unit controls the power supply based on the type of an internal organ determined by the internal organ determination unit.

A medical system according to the present invention includes: the above-described capsule medical device; and an external device provided outside of the subject, the external device including a capsule detection magnetic field detector configured to detect the magnetic field generated by the capsule detection magnetic field generation unit, a position and posture detection unit configured to detect at least one of the position and the posture of the capsule medical device based on the detection result of the capsule detection magnetic field detector, and a command information transmitting unit configured to transmit command information for controlling the power supply provided by the power supply unit. The state detection unit receives the command information transmitted from command information transmitting unit, and the control unit controls the power supply based on the command information received by the state detection unit. Advantageous Effects of Invention

According to the present invention, the power supply to the magnetic field generation unit from the power supply unit is controlled based on a detection result of the state of the capsule medical device; therefore, is becomes possible to alleviate electric power consumed for generating a magnetic field for detecting a position and a posture of the capsule medical device from the outside of a subject.

### Brief Description of Drawings

FIG. 1 is a schematic view illustrating an example of configuration of a medical system according to Embodiment 1-1.
FIG. 2 is a schematic sectional view illustrating an example of an internal structure of the capsule endoscope as illustrated in FIG. 1.
FIGS. 3 are perspective views illustrating an example of configuration of an operation input unit as illustrated in FIG. 1.
FIG. 4 is a schematic view illustrating movement of a capsule endoscope corresponding to input operation with the operation input unit as illustrated in FIGS. 3.
FIG. 5 is a schematic view for explaining arrangement of a guiding-magnetic field generation unit and a receiving unit in the guidance device as illustrated in FIG. 1.
FIG. 6 is a block diagram illustrating an example of configuration of a position and posture calculation unit as illustrated in FIG. 1.
FIG. 7 is a schematic view illustrating an example of display of a screen displayed on a display unit.
FIG. 8 is a flowchart illustrating operation of a medical system as illustrated in FIG. 1.
FIG. 9 is a flowchart illustrating operation of control of state detection and capsule detection magnetic field of a capsule endoscope.
FIG. 10 is a schematic view illustrating an example of configuration of a capsule detection magnetic field generation unit.
FIG. 11 is a schematic view illustrating an example of configuration of a guiding-magnetic field detector.
FIG. 12 is a flowchart illustrating operation of control of state detection and capsule detection magnetic field of a capsule endoscope according to Modification 1-1-2.
FIG. 13 is a flowchart illustrating operation of control of state detection and capsule detection magnetic field of a capsule endoscope according to Modification 1-1-3.
FIG. 14 is a flowchart illustrating operation of control of state detection and capsule detection magnetic field of a capsule endoscope according to Modification 1-1-4.
FIG. 15 is a schematic view for explaining a method for controlling power supply based on the size of the angle between the direction of the guiding-magnetic field and the magnetization direction of the permanent magnet.
FIG. 16 is a flowchart illustrating operation of control of state detection and capsule detection magnetic field of the capsule endoscope according to Modification 1-1-6.
FIG. 17 is a schematic sectional view illustrating an example of an internal structure of a capsule endoscope according to Embodiment 1-2.
FIG. 18 is a flowchart illustrating operation of control of state detection and capsule detection magnetic field of the capsule endoscope as illustrated in FIG. 17.
FIG. 19 is a flowchart illustrating operation of control of state detection and capsule detection magnetic field of the capsule endoscope according to Modification 1-2-1.
FIG. 20 is a flowchart illustrating operation of control of state detection and capsule detection magnetic field of a capsule endoscope according to Modification 1-2-2.
FIG. 21 is a figure illustrating an example of operation of a motion detector of the capsule endoscope.
FIG. 22 is a block diagram illustrating a configuration of a medical system according to Modification 1-2-5.
FIG. 23 is a schematic sectional view illustrating an example of an internal structure of the capsule endoscope as illustrated in FIG. 22.
FIG. 24 is a schematic sectional view illustrating an example of an internal structure of a capsule endoscope according to Embodiment 1-3.
FIG. 25 is a flowchart illustrating operation of control of state detection and capsule detection magnetic field of the capsule endoscope as illustrated in FIG. 24.
FIG. 26 is a flowchart illustrating operation of control of state detection and capsule detection magnetic field of a capsule endoscope according to Modification 1-3-1.
FIG. 27 is a flowchart illustrating operation of control of state detection and capsule detection magnetic field of a capsule endoscope according to Embodiment 1-4.
FIG. 28 is a schematic view for explaining a method of detecting the state of the capsule endoscope according to Embodiment 1-4.
FIG. 29 is a schematic sectional view illustrating an example of an internal structure of a capsule endoscope provided in a medical system according to Embodiment 2-1.
FIG. 30 is a flowchart illustrating operation of control of state detection and capsule detection magnetic field of the capsule endoscope as illustrated in FIG. 29.
FIG. 31 is a flowchart illustrating operation of control of state detection and capsule detection magnetic field of a capsule endoscope according to Modification 2-1-1.
FIG. 32 is a schematic view illustrating a configuration and an operation of a distance acquiring unit according to Modification 2-1-3.
FIG. 33 is a schematic sectional view illustrating an example of an internal structure of a capsule endoscope provided in a medical system according to Embodiment 2-2.
FIG. 34 is a flowchart illustrating operation of control of state detection and capsule detection magnetic field of the capsule endoscope as illustrated in FIG. 33.
FIG. 35 is a block diagram illustrating a configuration of a medical system according to Embodiment 3-1.
FIG. 36 is a schematic sectional view illustrating an internal configuration of the capsule endoscope as illustrated in FIG. 35.
FIG. 37 is a flowchart illustrating operation of control of state detection and capsule detection magnetic field of the capsule endoscope as illustrated in FIG. 35.
FIG. 38 is a block diagram illustrating a configuration of a medical system according to Embodiment 3-3.
FIG. 39 is a schematic sectional view illustrating an example of an internal structure of the capsule endoscope as illustrated in FIG. 38.
FIG. 40 is a schematic sectional view illustrating an internal configuration of a capsule endoscope according to Modification 4.
FIGS. 41 are schematic views illustrating examples of displays of in-vivo images according to Modification 4.
FIG. 42 is a schematic sectional view illustrating an internal configuration of a capsule endoscope according to Modification 4.
FIG. 43 is a schematic sectional view illustrating an internal configuration of a capsule endoscope according to Modification 5.
FIG. 44 is a schematic view illustrating a capsule endoscope drifting about in an internal organ into which liquid has been introduced.
FIGS. 45 are schematic views for explaining a control method of an image-capturing frame rate of the capsule endoscope according to Modification 5.

### Description of Embodiments

Hereinafter, a capsule medical device and a medical system according to embodiments of the present invention will be explained with reference to drawings. In the explanation below, a capsule endoscope serving as a capsule medical device introduced from the mouth of the subject into the subject and drifts about in a liquid accumulated in the stomach of a subject is used for example, but the present invention is not limited by the embodiments. More specifically, the present invention can be used for various kinds of capsule medical devices, for example, a capsule endoscope that moves through the lumens from the esophagus to the anus of the subject, a capsule endoscope introduced together with isotonic solution from the anus, and a capsule medical device delivering medical agent and the like into the subject.

In the present application, magnetic field for magnetically guiding a capsule medical device may also be referred to as guiding-magnetic field.

In the explanation below, each drawing is nothing but schematically illustrating the relationship of the shape, the size, and the position in such a manner that the contents of the present invention can be understood. Therefore, the present invention is not limited to the relationship of the shape, the size, and the position as illustrated in each drawing, for example. It should be noted that, in the description of the drawings, the same portions are denoted with the same reference numerals.

### (Embodiment 1-1)

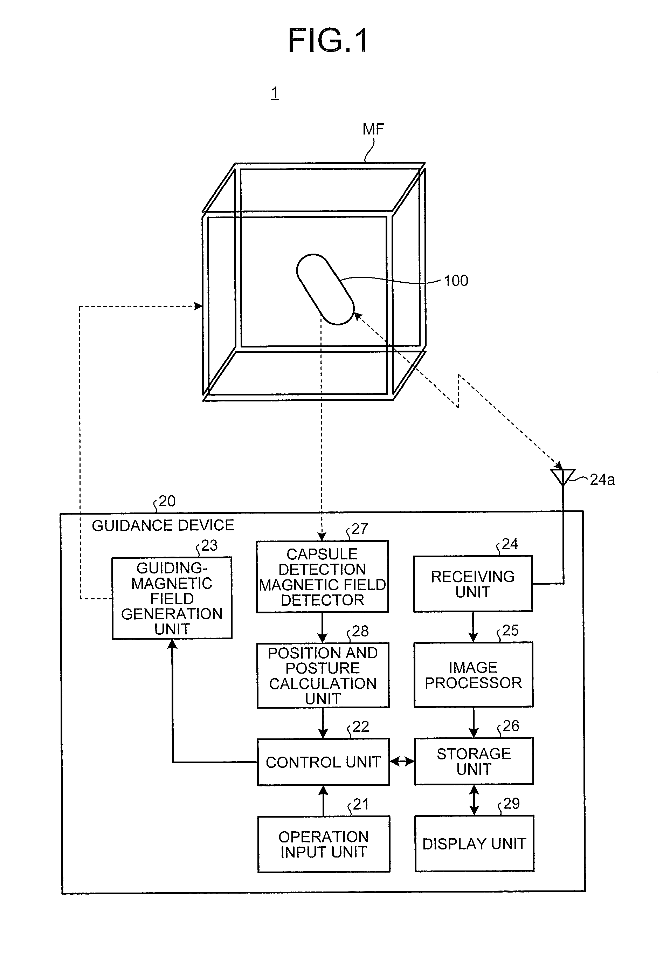

FIG. 1 is a schematic view illustrating an example of configuration of a medical system according to Embodiment 1-1 of the present invention. As illustrated in FIG. 1, a medical system 1 according to Embodiment 1-1 is a capsule medical device that is introduced into the body cavity of a subject, and includes a capsule endoscope 100 configured to acquire image information within the subject and wirelessly transmit the image information, and also includes a guidance device 20 configured to magnetically guide the capsule endoscope 100 by generating a three-dimensional magnetic field (guiding-magnetic field) MF around the subject. The guidance device 20 includes an operation input unit 21, a control unit 22 for controlling operation of each unit of the guidance device 20, a guiding-magnetic field generation unit 23 for generating the guiding-magnetic field MF, a receiving unit 24 for receiving image information wirelessly transmitted from the capsule endoscope 100, an image processor 25 for performing predetermined image processing on image information received by the receiving unit 24, a storage unit 26, a capsule detection magnetic field detector 27 for detecting capsule detection magnetic field (explained later) generated by the capsule endoscope 100, a position and posture calculation unit 28 for calculating the position and the posture of the capsule endoscope 100 based on the detected capsule detection magnetic field, and a display unit 29.

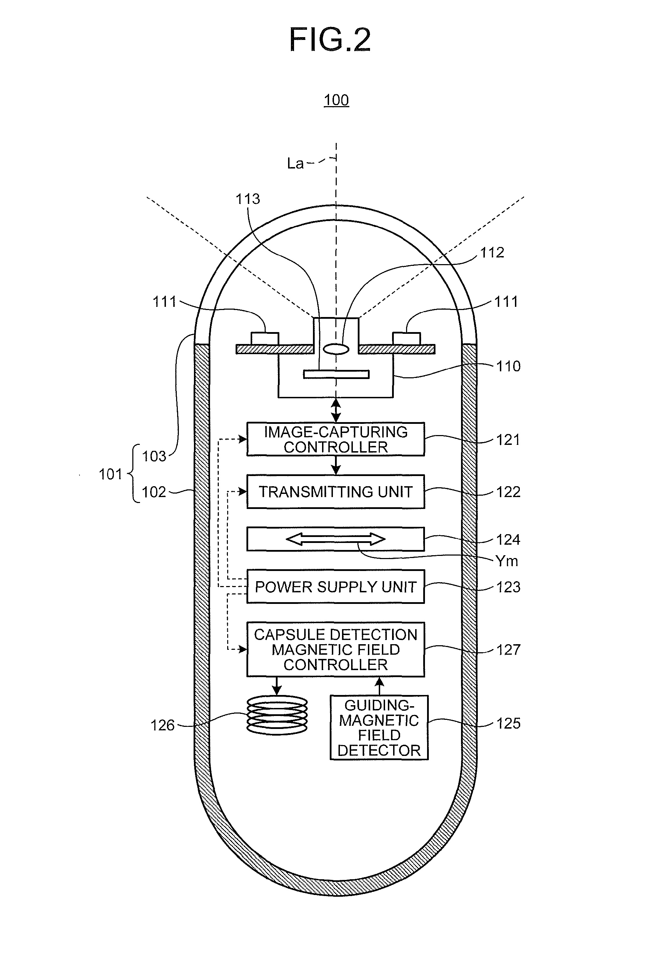

FIG. 2 is a schematic sectional view illustrating an example of an internal structure of the capsule endoscope 100 as illustrated in FIG. 1. As illustrated in FIG. 2, the capsule endoscope 100 includes a capsule-shaped casing 101 which is an exterior formed to be of the size so that it can be easily introduced into the inside of an internal organ of a subject and an image-capturing unit 110 provided at one of the end portions of the capsule-shaped casing 101 to acquire image-captured signal by capturing images of the subject. In addition, the capsule endoscope 100 includes an image-capturing controller 121 for controlling operation of the image-capturing unit 110 and generating image information about the inside of the subject by performing predetermined signal processing on the image-captured signal acquired by the image-capturing unit 110, a transmitting unit 122 wirelessly transmitting the image information generated by the image-capturing controller 121, a power supply unit 123 providing electric power to each unit of the capsule endoscope 100, a permanent magnet 124 serving as a magnetic field response unit for responding to the guiding-magnetic field MF, a guiding-magnetic field detector 125 for detecting the guiding-magnetic field MF, a capsule detection magnetic field generation unit 126, and a capsule detection magnetic field controller 127.

The capsule-shaped casing 101 is an exterior case formed to be of the size so that the capsule-shaped casing 101 can be introduced into the inside of the internal organ of the subject, and is made by shielding the opening end of the casing (cylindrical casing) 102, one end of the cylindrical shape is shielded, with a dome-shaped casing 103. The dome-shaped casing 103 is a dome-shaped optical member which is transparent to light of a predetermined wavelength band such as visible light. The cylindrical casing 102 is a colored casing which is substantially opaque to the visible light. The capsule-shaped casing 101 formed by the cylindrical casing 102 and the dome-shaped casing 103 include, therein in liquid-tight manner, the image-capturing controller 121, the transmitting unit 122, the power supply unit 123, the permanent magnet 124, the guiding-magnetic field detector 125, the detection magnetic field generation unit 126, and the detection magnetic field controller 127.

The image-capturing unit 110 includes an illumination unit 111 such as LED, an optical system 112 such as a condenser lens, and an image-capturing device 113 such as a CMOS image sensor or a CCD. The illumination unit 111 emits illumination light such as white light to an image-capturing vision field of the image-capturing device 113, and illuminates a subject within image-capturing vision field through the dome-shaped casing 103. The optical system 112 condenses the reflected light from the image-capturing vision field onto the image-capturing surface of the image-capturing device 113, and forms an image (which may also be hereinafter referred to as an in-vivo image) within the subject of the image-capturing vision field on the image-capturing surface of the image-capturing device 113. The image-capturing device 113 receives the reflected light from the image-capturing vision field via the image-capturing surface and performs photoelectric conversion processing in the light signal thus received, thus generating image-captured signal corresponding to an image-capturing vision field.

The image-capturing controller 121 controls operation of the image-capturing unit 110. More specifically, the image-capturing controller 121 operates the image-capturing device 113 with a predetermined image-capturing frame rate, and in synchronization with the image-capturing frame rate, the image-capturing controller 121 causes the illumination unit 111 to emit light. The image-capturing controller 121 performs A/D conversion, and other predetermined signal processing on the image-captured signal acquired by the image-capturing unit 110, thus generating image data. Further, in accordance with the strength of the image-captured signal given by the image-capturing unit 110, the image-capturing controller 121 generates light adjustment information indicating an appropriate amount of light emission of the illumination unit 111, and controls the illumination unit 111 based on the light adjustment information. More specifically, when the strength of the image-captured signal is high (when the reflected light from the image-capturing target is strong, i.e., the distance from the image-capturing target is close), light adjustment information for reducing the amount of light emission is generated. When the strength of the image-captured signal is low (when the reflected light from the image-capturing target is weak, i.e., the distance from the image-capturing target is far), light adjustment information for increasing the amount of light emission is generated. This light adjustment information is associated with the corresponding image data by the image-capturing controller 121, and is wirelessly transmitted together with image data as related information related to the image data.

The transmitting unit 122 includes an antenna (not illustrated), and acquires the image data and the related information generated by the image-capturing controller 121 and performs the modulation processing, and transmits the image data and the related information generated by the image-capturing controller 121 to the outside via the antenna in order.

The power supply unit 123 is achieved with a battery unit such as a button-shaped battery or a capacitor, and a switch unit such as a magnetic switch. The power supply unit 123 switches the ON/OFF state of itself by the magnetic field that is applied from the outside, and when the ON/OFF state is in the ON state, the power supply unit 123 provides, as necessary, the electric power of the battery unit to each unit of the capsule endoscope 100. When the ON/OFF state is in the OFF state, the power supply unit 123 stops the electric power provided to each unit of the capsule endoscope 100.

The permanent magnet 124 is to enable magnetic guidance of the capsule endoscope 100 using the magnetic field MF generated by the guidance device 20. The permanent magnet 124 is arranged, in a fixed manner, inside of the capsule-shaped casing 101 so that the magnetization direction Ym of itself is inclined with respect to the longitudinal axis La of the capsule endoscope 100. In Embodiment 1-1, the permanent magnet 124 is arranged so that the magnetization direction Ym is perpendicular to the longitudinal axis La. The permanent magnet 124 operates so as to follow the guiding-magnetic field MF that is applied from the outside, and as a result, this achieves the magnetic guidance of the capsule endoscope 100 by the guidance device 20. More specifically, this achieves movement of the capsule endoscope 100 to a desired position.

The guiding-magnetic field detector 125 is a form of state detection unit for detecting the state under which the capsule endoscope 100 is placed, and detects at least one of the strength, the direction, and the gradient of the guiding-magnetic field MF generated by the guidance device 20 and applied to the capsule endoscope 100. In Embodiment 1-1, the guiding-magnetic field detector 125 detects the strength of the guiding-magnetic field MF. It should be noted that the strength of the guiding-magnetic field MF can be detected using, for example, a three-axis magnetic field sensor capable of detecting the magnetic field strengths in three axes (X axis, Y axis, Z axis) directions.

The capsule detection magnetic field generation unit (which may also be hereinafter simply referred to as a detection magnetic field generation unit) 126 generates capsule detection magnetic field with which the position and/or the posture of the capsule endoscope 100 is detected by the guidance device 20. More specifically, the detection magnetic field generation unit 126 is achieved with a coil that receives the electric power provided from the power supply unit 123 and generates the alternate current magnetic field.

The capsule detection magnetic field controller (which may also be hereinafter simply referred to as a detection magnetic field controller) 127 controls the electric power which the power supply unit 123 provides to the detection magnetic field generation unit 126 based on the detection result of the guiding-magnetic field detector 125, so that the strength of the detection magnetic field generated by the detection magnetic field generation unit 126 is changed. More specifically, the capsule detection magnetic field controller 127 controls the strength of the electric current provided to the detection magnetic field generation unit 126.

Subsequently, the configuration of the guidance device 20 will be explained in detail.

The operation input unit 21 is achieved with an input device such as an operation device such as a joy stick, various kinds of buttons, switches, and a keyboard, and a pointing device such as a mouse and a touch panel, and inputs various kinds of information into the control unit 22 in accordance with input operation performed by a user such as a doctor. Information which is input from the operation input unit 21 into the control unit 22 includes command information for the control unit 22, patient information and examination information about a subject, guiding command information for magnetically guiding the capsule endoscope 100. Among them, the patient information about the subject is identification information for identifying the subject, and is, for example, the patient name of the subject, the patient ID, the date of birth, sex, age, and the like. The examination information about the subject is identification information for identifying the examination for observing the inside of the digestive tract by introducing the capsule endoscope 100 into the inside of the digestive tract of the subject, and is, for example, the examination ID, the examination date, and the like. It should be noted that the patient information and the examination information are input using, for example, a keyboard and a mouse. The guiding command information is information for commanding, e.g., the direction in which the capsule endoscope 100 is moved in the subject, the strength of the force applied when the capsule endoscope 100 is moved, and the posture of the capsule endoscope 100. Such guiding command information is input using, for example, a joy stick. Alternatively, in order to accelerate movement of the capsule endoscope 100, a button corresponding to a mode for temporarily strongly urging the capsule endoscope 100 is separately provided, and while this button is pressed down, guiding command information indicating the mode may be continuously input.

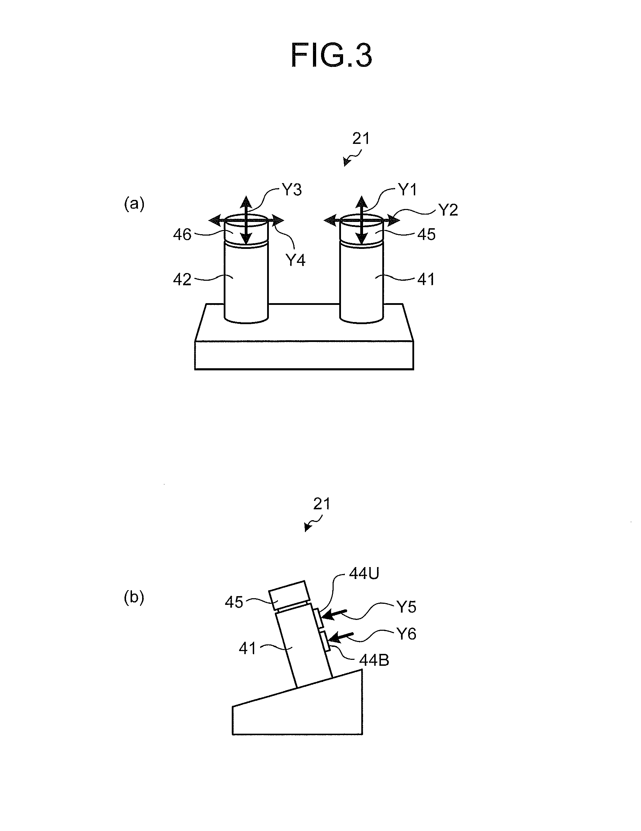

FIGS. 3 are perspective views illustrating an example of configuration of the operation input unit 21. For example, as illustrated in FIG. 3(a), the operation input unit 21 is constituted by an operation input device that includes two joy sticks 41, 42. The joy sticks 41, 42 are used to operate, in a three dimensional manner, the capsule endoscope 100 by magnetic guidance, and can be inclined and operated in the up/down and right/left directions in the drawings.

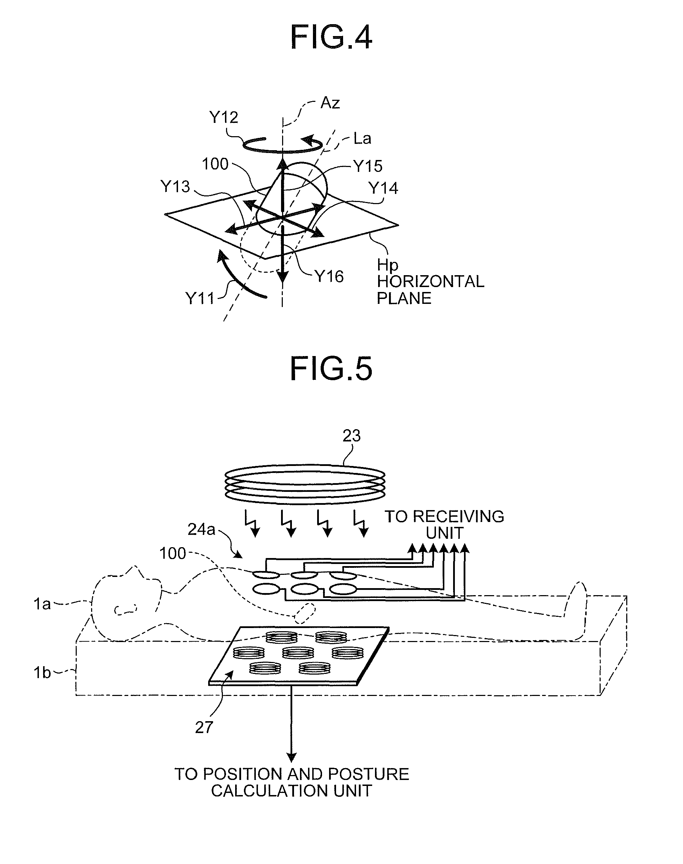

As illustrated in FIG. 3(a), when the joy stick 41 is tilted and moved in the up/down direction indicated by arrow Y1, guiding command information corresponding to tilting guidance for swinging the capsule endoscope 100 so that the forward end of the capsule endoscope 100 passes the vertical axis Az as indicated by arrow Y11 of FIG. 4 is input into the control unit 22.

As illustrated in FIG. 3(a), when the joy stick 41 is tilted and moved in the right/left direction indicated by arrow Y2, guiding command information corresponding to rotation guiding direction for rotating the capsule endoscope 100 so that it rotates about the vertical axis Az as indicated by arrow Y12 of FIG. 4 is input into the control unit 22.

As illustrated in FIG. 3(a), when the joy stick 42 is tilted and moved in the up/down direction indicated by arrow Y3, guiding command information corresponding to horizontal backward guiding or horizontal forward guiding for advancing in a direction in which the longitudinal axis La of the capsule endoscope 100 is projected on the horizontal plane Hp as indicated by arrow Y13 of FIG. 4 is input into the control unit 22.

As illustrated in FIG. 3(a), when the joy stick 42 is tilted and moved in the right/left direction indicated by arrow Y4, guiding command information corresponding to horizontal right guiding or horizontal left guiding for advancing the capsule endoscope 100 on the horizontal plane Hp in a direction perpendicular to the direction in which the longitudinal axis La is projected on the horizontal plane Hp as indicated by arrow Y14 of FIG. 4 is input into the control unit 22.

As illustrated in FIG. 3(b), an up button 44U and a down button 44B are provided on the back surface of the joy stick 41. When the up button 44U is pressed in a direction indicated by arrow Y5, guiding command information for guiding the capsule endoscope 100 to the upper side is input into the control unit 22 as indicated by arrow Y15 of FIG. 4. When the down button 44B is pressed in a direction indicated by arrow Y6, guiding command information for guiding the capsule endoscope 100 to the lower side is input into the control unit 22 as indicated by arrow Y16 of FIG. 4.

At the upper portion of the joy stick 41, a capture button 45 is provided. When the capture button 45 is pressed, command information for capturing an in-vivo image displayed on the display unit 29 is input into the control unit 22. At the upper portion of the joy stick 42, an approach button 46 is provided. When the approach button 46 is pressed, guiding command information for guiding the capsule endoscope 100 so that the side of the image-capturing unit 110 approaches the image-capturing target (e.g., the inner wall of the digestive tract) is input into the control unit 22.

The control unit 22 controls each unit of the guidance device 20 based on various kinds of information which are input from the operation input unit 21. More specifically, the control unit 22 controls the guiding-magnetic field generation unit 23 so as to generate the guiding-magnetic field MF for guiding the capsule endoscope 100 to a user desired the position and the posture based on the guiding command information which is input from the operation input unit 21 and the position and the posture information about the capsule endoscope 100 calculated by the position and posture calculation unit 28.

The guiding-magnetic field generation unit 23 is achieved using, for example, multiple coils and the like.

A power supply unit, not illustrated, provides electric power to these coils, so that the magnetic fields generated by the coils are combined, and the three-dimensional guiding-magnetic field MF is generated. The strength, the direction, and the gradient of the guiding-magnetic field MF can be appropriately changed by adjusting the electric power provided to the coils under the control of the control unit 22. As illustrated in FIG. 5, the guiding-magnetic field generation unit 23 is arranged, for example, above the examination target region (for example, an abdominal region) of a subject 1a. It should be noted that the guiding-magnetic field generation unit 23 may be constituted by multiple permanent magnets, and in this case, control is performed to cause only necessary permanent magnets to come in proximity to the subject.

For example, as illustrated in FIG. 5, the receiving unit 24 includes multiple antennas 24a pasted to the body surface of the subject 1a, and receives image information wirelessly transmitted via the multiple antennas 24a from the capsule endoscope 100. More specifically, the receiving unit 24 selects an antenna of which received strength is the highest from among the multiple antennas 24a, and performs demodulation processing and the like and the like on the wireless signal received via the selected antenna, thus extracting image data corresponding to an in-vivo image of the subject 1a.

The image processor 25 generates image data for display, by performing predetermined image processing such as white balance processing, demosaicing, gamma conversion, etc., smoothing (noise removal, etc.) on the image data received by the receiving unit 24.

The storage unit 26 is achieved with a storage medium for saving information to flash memory, a hard disk, or the like in a rewritable manner. The storage unit 26 stores not only image data of the in-vivo image of the subject 1a captured by the capsule endoscope 100 but also information such as various kinds of programs and various kinds of parameters with which the control unit 22 controls each unit of the guidance device 20.

For example, the capsule detection magnetic field detector 27 is achieved with multiple coils arranged within a bed 1b on which the subject 1a lies down as illustrated in FIG. 5. Each of these coils detects the capsule detection magnetic field (alternate current magnetic field) that is generated by the detection magnetic field generation unit 126 of the capsule endoscope 100.

The position and posture calculation unit 28 calculates the position and the posture of the capsule endoscope 100 from the alternate current magnetic field detected by each coil of the capsule detection magnetic field detector 27.

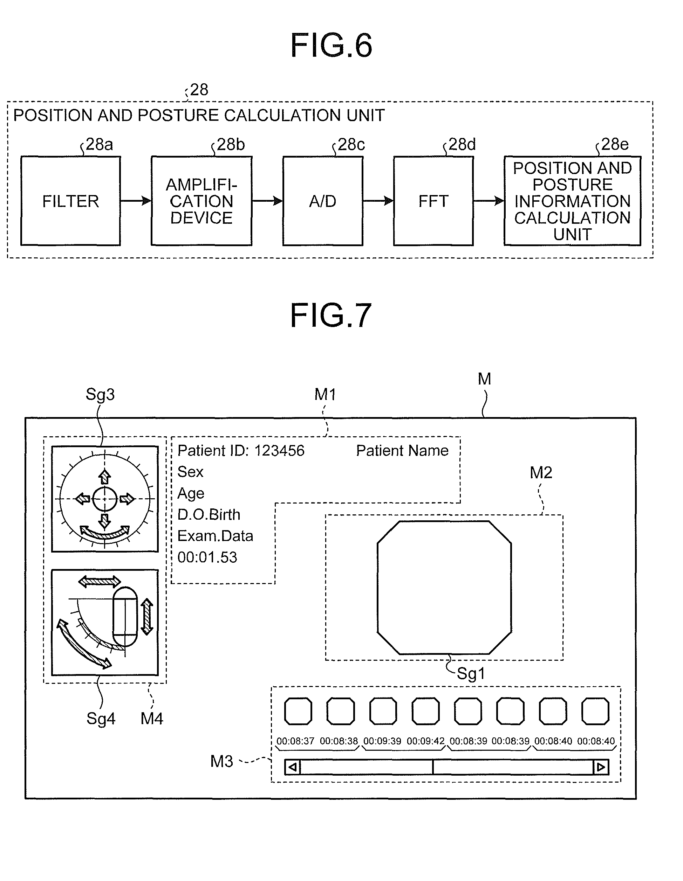

FIG. 6 is a block diagram illustrating an example of configuration of the position and posture calculation unit 28. As illustrated in FIG. 6, the position and posture calculation unit 28 includes a filter 28a for removing noises by predetermined filter processing on a signal given by the capsule detection magnetic field detector 27 (detection signal of the alternate current magnetic field detected by each coil), an amplification device 28b, an A/D converter (A/D) 28c for generating detection data by applying A/D conversion processing on the detection signal of the alternate current magnetic field, an FFT calculator (FFT) 28d for applying Fast Fourier Transformation (FFT) processing on the detection data, and a position and posture information calculation unit 28e for performing calculation for acquiring the position and the posture information about the capsule endoscope 100 based on the detection data on which the FFT processing is performed.

The display unit 29 includes various kinds of displays such as liquid crystal and organic EL, and displays, on a screen, the in-vivo image of the subject 1a in real time, and displays, on the screen, guiding command information which is input from the operation input unit 21, and various other kinds of information.

FIG. 7 is a schematic view illustrating an example of display of a screen M displayed on the display unit 29. In this menu screen M, each subject information such as the patient name of the subject 1a, the patient ID, the date of birth, sex, and age, are displayed in a region M1 at the upper left corner, and a living body image Sg1 captured by the image-capturing unit 110 is displayed in a region M2 in the center. In a region M3 below the region M2, each image captured in response to pressing operation of the capture button 45 as well as a capturing time are displayed in a reduced manner. In the region M4 at the left, a posture figure Sg3 in the vertical plane is displayed, and a posture figure Sg4 in the horizontal plane is displayed, serving as a posture figure of the capsule endoscope 100. The posture of the capsule endoscope 100 displayed in each posture figure Sg3, Sg4 illustrates the posture corresponding to the guiding command information of the operation input unit 21. In Embodiment 1, the amount of input by the operation input unit 21 is reflected in the force of guiding. Therefore, the posture of the capsule endoscope 100 displayed may be considered to be substantially the same as the actual posture of the capsule endoscope 100, and the user's maneuverability is improved. It should be noted that, in the posture figures Sg3, Sg4, a direction in which the capsule endoscope 100 can be guided is indicated by an arrow, and when operation input is given in whichever guiding direction, the display color of the arrow corresponding to the direction which has been input may be changed, so that the user's maneuverability is improved.

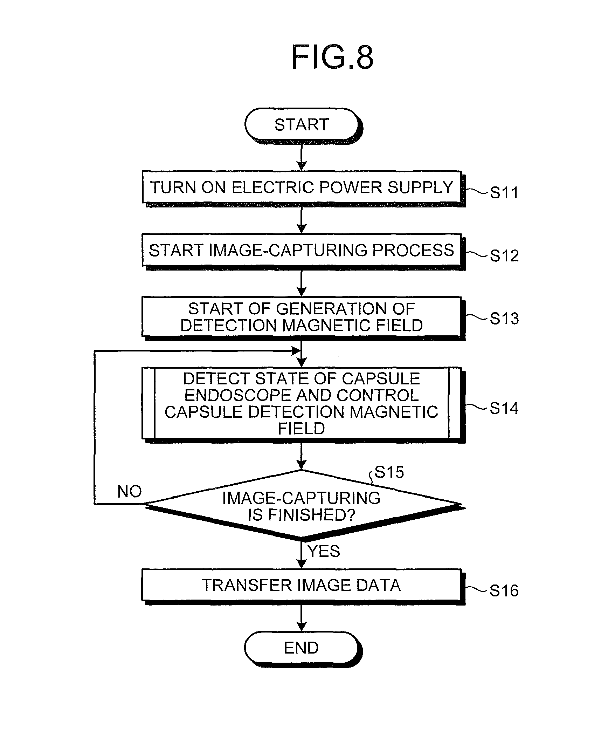

Subsequently, the operation of the capsule endoscope 100 will be explained. FIG. 8 is a flowchart illustrating operation of the medical system 1.

When the capsule endoscope 100 is turned on in step S11, the image-capturing unit 110 starts image-capturing process in step S12. In step S13, the detection magnetic field generation unit 126 starts generation of the capsule detection magnetic field. The operations in steps S12, S13 are not limited to the order described above. The operations in steps S12, S13 may be performed in the reversed order, or may be executed at a time, as necessary.

In step S14 subsequent thereto, the detection magnetic field controller 127 detects the state under which the capsule endoscope 100 is placed, and in accordance with the detection result, the detection magnetic field controller 127 controls the capsule detection magnetic field.

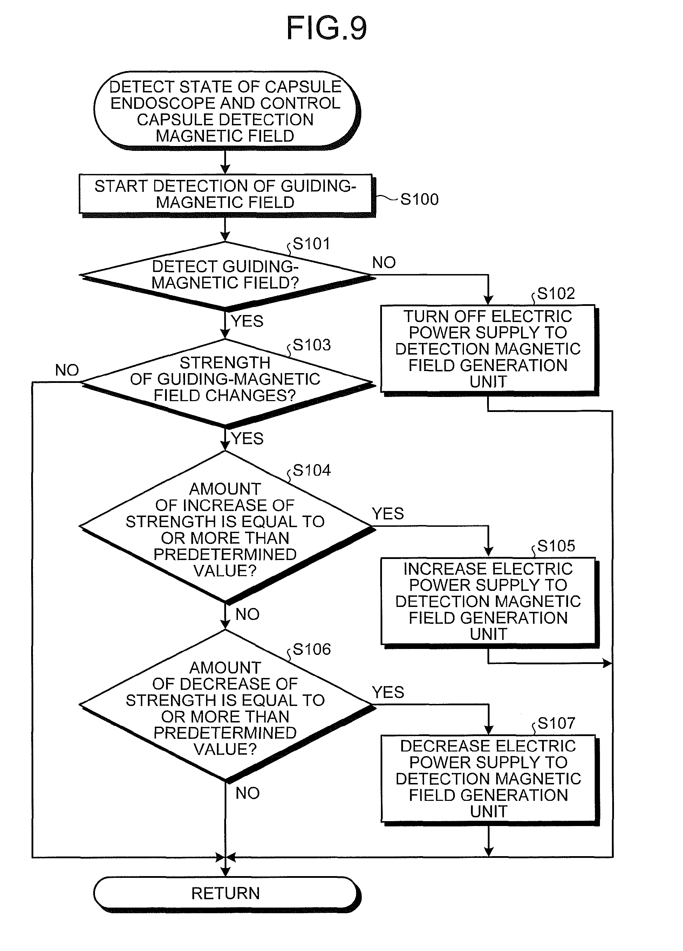

FIG. 9 is a flowchart illustrating the details of operation in step S14. In Embodiment 1, the strength of the guiding-magnetic field MF that is applied to the capsule endoscope 100 is detected as the state of the capsule endoscope 100.

First, in step S100, the guiding-magnetic field detector 125 starts the detection of the guiding-magnetic field MF generated by the guidance device 20.

In step S101 subsequent thereto, the detection magnetic field controller 127 determines whether the guiding-magnetic field MF is detected or not, from the output signal of the guiding-magnetic field detector 125. When the guiding-magnetic field MF is not detected (step S101: No), more specifically, when the guidance device 20 does not guide the capsule endoscope 100, the detection magnetic field controller 127 turns off the power supply provided to the detection magnetic field generation unit 126 (step S102). This is because, when the guidance device 20 does not guide the capsule endoscope 100, it is not necessary to acquire the position and the posture of the capsule endoscope 100, and therefore, it is not necessary to generate the capsule detection magnetic field. At this occasion, the power supply provided to the detection magnetic field generation unit 126 may not be completely turned off, and the power supply may be reduced. In this case, the guidance device 20 can acquire the position and the posture information about the capsule endoscope 100 which is simply reference information (which means that the accuracy is not so much high). Thereafter, the operation of the capsule endoscope 100 returns back to the main routine.

On the other hand, when the guiding-magnetic field MF is detected (step S101: Yes), the detection magnetic field controller 127 determines whether the strength of the guiding-magnetic field MF has been changed or not, from the output signal of the guiding-magnetic field detector 125 (step S103). When the strength of the guiding-magnetic field MF is changed (step S103: Yes), the guiding-magnetic field detector 125 determines whether the amount of increase in the strength of the guiding-magnetic field MF is equal to or more than a predetermined value (step S104).

When the amount of increase in the strength of the guiding-magnetic field MF is determined to be equal to or more than the predetermined value (step S104: Yes), the detection magnetic field controller 127 increases the power supply provided to the detection magnetic field generation unit 126 (step S105). More specifically, the electric current flowing through the detection magnetic field generation unit 126 is increased. Accordingly, the strength of the capsule detection magnetic field is increased.

In this case, rapid increase in the strength of the guiding-magnetic field MF is determined that the guidance device 20 turns ON the mode for temporarily strongly urging the capsule endoscope 100 in order to forcibly move the capsule endoscope 100. It should be noted that such case occurs when, for example, it is restrained by residual in the pleats of the inner wall and the lumens in the internal organs in the subject 1a. In this case, due to the increase of the strength of the guiding-magnetic field MF, the SN ratio of the detection signal of the capsule detection magnetic field generated by the capsule endoscope 100 may be deteriorated. Moreover, the capsule endoscope 100 moves at a high speed, and therefore, it is highly necessary to improve the accuracy of the detection position of the capsule endoscope 100. Accordingly, in the capsule endoscope 100, the strength of the capsule detection magnetic field is increased, so that the deterioration of the SN ratio of the detection signal in the guidance device 20 can be suppressed. As a result, the guidance device 20 can detect the position and the posture of the capsule endoscope 100 with a high degree of accuracy, and the guiding-magnetic field MF for guiding the position and the posture of the capsule endoscope 100 can be generated in proximity to the capsule endoscope 100 with a higher degree of accuracy and efficiency. It should be noted that when the power supply provided to the detection magnetic field generation unit 126 is already increased, it is sufficient to maintain the level of the electric power in step S105. Thereafter, the operation of the capsule endoscope 100 returns back to the main routine.

In step S104, when the amount of increase in the strength of the guiding-magnetic field MF is smaller than a predetermined value (step S104: No), the detection magnetic field controller 127 determines whether the amount of decrease in the strength of the guiding-magnetic field MF is equal to or more than a predetermined value, from the output signal of the guiding-magnetic field detector 125 (step S106).

When the amount of decrease in the strength of the guiding-magnetic field MF is determined to be equal to or more than the predetermined value (step S106: Yes), the detection magnetic field controller 127 decreases the electric power given to the detection magnetic field generation unit 126 (step S107). More specifically, the electric current flowing through the detection magnetic field generation unit 126 is reduced. Accordingly, the strength of the capsule detection magnetic field is decreased.

In this case, rapid great decrease in the strength of the guiding-magnetic field MF is determined that the guidance device 20 turns OFF state the mode for temporarily strongly urging the capsule endoscope 100. In this case, it is not necessary to so much increase the accuracy of detection by the guidance device 20 for detecting the position and the posture of the capsule endoscope 100. Therefore, by weakening the generation strength of the capsule detection magnetic field, the electric power that is consumed by the capsule endoscope 100 can be suppressed. Thereafter, the operation of the capsule endoscope 100 returns back to the main routine.

On the other hand, when the strength of the guiding-magnetic field MF does not change in step S103 (step S103: No), or when the amount of decrease in the strength of the guiding-magnetic field MF is less than the predetermined value in step S106 (step S106: No), then the operation of the capsule endoscope 100 returns back to the main routine.

In step S15 of FIG. 8, the capsule endoscope 100 determines whether the image-capturing within the subject is to be terminated or not. For example, when a predetermined period of time has passed since the capsule endoscope 100 is turned on, and the amount of electric power accumulated in the power supply unit 123 is equal to or less than the predetermined value, then the image-capturing within the subject is determined to be terminated. In this case (step S15: Yes), the operation of the medical system 1 proceeds to step S16. On the other hand, when the image-capturing within the subject is determined not to be terminated (step S15: No), the operation of the medical system 1 returns back to step S14.

In step S16, the image data accumulated in the storage unit 26 of the guidance device 20 are transferred to a diagnosis image display apparatus such as a work station connected via a cable and the like, and image processing is further applied, and the user interprets and diagnoses the image. It should be noted that the storage unit 26 of the guidance device 20 is constituted using a portable recording medium, and by setting this recording medium to a reading device provided in the diagnosis image display apparatus, the image data may be transferred.

As described above, according to Embodiment 1-1, the power supply provided to the detection magnetic field generation unit 126 is controlled based on the strength of the guiding-magnetic field MF that is applied to the capsule endoscope 100, and therefore, while the capsule detection magnetic field is generated in accordance with the necessity of detection of the capsule endoscope 100, the electric power that is consumed by the capsule endoscope 100 can be suppressed.

In step S105 explained above, the power supply applied to the detection magnetic field generation unit 126 is increased, but when the power supply provided to the detection magnetic field generation unit 126 is already turned off until then, it may be turned on. In step S107 explained above, the power supply provided to the detection magnetic field generation unit 126 is reduced. Alternatively, the power supply may be turned off.

### (Modification 1-1-1)

Subsequently, Modification 1-1-1 of Embodiment 1-1 will be explained.

In Embodiment 1-1 explained above, the increase or the decrease of the power supply provided to the detection magnetic field generation unit 126 is controlled by adjusting the electric current flowing through the detection magnetic field generation unit 126. However, when the power supply provided to the detection magnetic field generation unit 126 can be increased or decreased in total, the other methods may also be used. For example, in a case where the electric power is provided from the power supply unit 123 to the detection magnetic field generation unit 126 in an intermittent manner (pulse manner), the interval according to which the electric power is provided (pulse interval) is reduced (i.e., the frequency is increased) when the power supply is increased, the interval according to which the electric power is provided (pulse interval) is increased (i.e., the frequency is decreased) when the power supply is decreased.

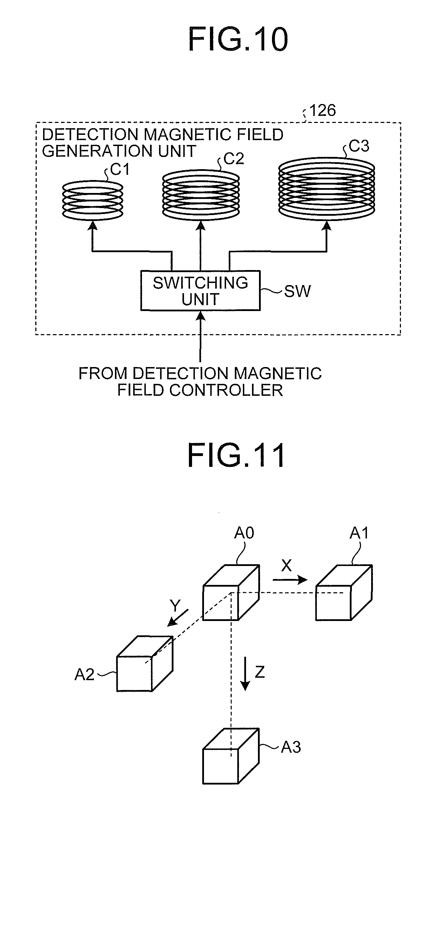

Alternatively, in accordance with the increase and the decrease of the power supply, the coil for generating the detection magnetic field may be changed. For example, as illustrated in FIG. 10, the detection magnetic field generation unit 126 is constituted by multiple coils C1 to C3 of which impedances are different from each other, and a switching unit SW. In accordance with the control of the detection magnetic field controller 127, the switching unit SW selects, from the coils C1 to C3, a coil to which the power supply of the power supply unit 123 is provided. More specifically, when the power supply is to be increased, a high impedance coil is selected, and when the power supply is to be decreased, a low impedance coil is selected.

### (Modification 1-1-2)

Subsequently, Modification 1-1-2 of Embodiment 1-1 will be explained.

In control operation of the state detection and capsule detection magnetic field of the capsule endoscope (step S14), the power supply provided to the detection magnetic field generation unit 126 may be controlled based on characteristics other than the strength of the guiding-magnetic field MF.

For example, when the guiding-magnetic field generation unit 23 of the guidance device 20 generates gradient magnetic field as the guiding-magnetic field MF, the capsule endoscope 100 causes the guiding-magnetic field detector 125 to detect the change of the gradient magnetic field of the guiding-magnetic field MF, and based on the change of the gradient of the guiding-magnetic field MF, the detection magnetic field controller 127 may be caused to control the power supply provided to the detection magnetic field generation unit 126.

In this case, for example, as illustrated in FIG. 11, the guiding-magnetic field detector 125 is achieved by arranging, in three-axis directions, four three-axis magnetic field sensors A0, A1, A2, A3 each capable of detecting the magnetic field strength in three-axis (X axis, Y axis, Z axis) directions.

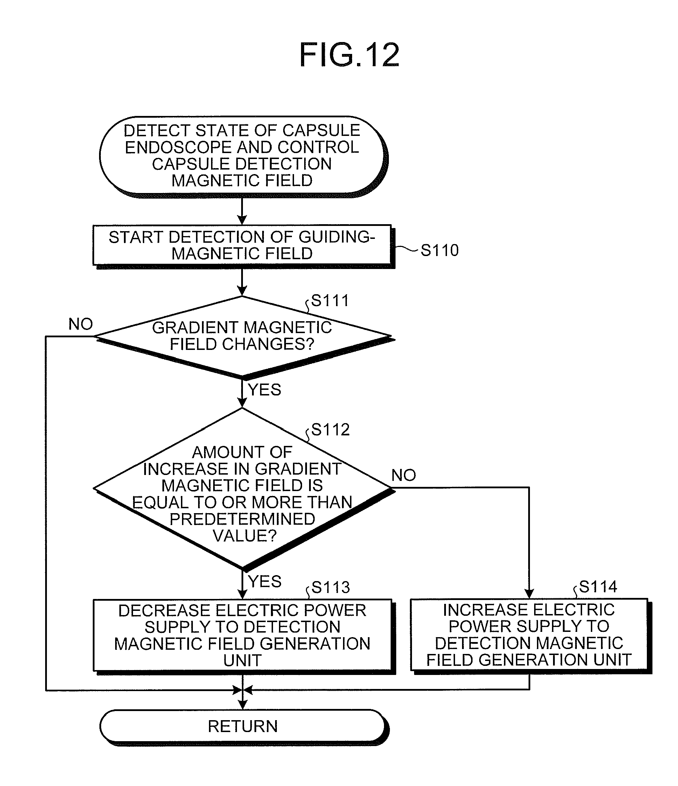

FIG. 12 is a flowchart illustrating control operation of the state detection and capsule detection magnetic field of the capsule endoscope according to Modification 1-1-2 (step S14).

First, in step S110, the guiding-magnetic field detector 125 starts the detection of the guiding-magnetic field MF (gradient magnetic field).

In step S111 subsequent thereto, the detection magnetic field controller 127 determines whether the gradient magnetic field is changed or not from the output signal of the guiding-magnetic field detector 125. When the gradient magnetic field changes (step S111: Yes), the operation of the capsule endoscope 100 proceeds to step S112. On the other hand, when the gradient magnetic field does not change (step S111: No), the operation of the capsule endoscope 100 returns back to the main routine.

In step S112, the detection magnetic field controller 127 determines whether the amount of increase of the gradient magnetic field is equal to or more than a predetermined value. When the amount of increase in the gradient magnetic field is determined to be equal to or more than the predetermined value (step S112: Yes), the detection magnetic field controller 127 decreases the power supply provided to the detection magnetic field generation unit 126 (step S113). Accordingly, the strength of the capsule detection magnetic field is decreased.

In this case, when the amount of increase in the gradient magnetic field is determined to be equal to or more than the predetermined value, this means that the guidance device 20 can reliably execute the guiding of the capsule endoscope 100 with the gradient magnetic, and it can be determined that it is not necessary for the guidance device 20 to detect the position and the posture of the capsule endoscope 100 with a high degree of accuracy again. Therefore, in this case, the generation strength of the detection magnetic field is decreased, and the electric power that is consumed by the capsule endoscope 100 can be suppressed. Thereafter, the operation of the capsule endoscope 100 returns back to the main routine.

On the other hand, when the amount of increase in the gradient magnetic field is determined to be less than the predetermined value (step S112: No), the detection magnetic field controller 127 increases the power supply provided to the detection magnetic field generation unit 126 (step S114). Accordingly, the strength of the capsule detection magnetic field is increased.

In this case, when the amount of increase in the gradient magnetic field is determined to be less than the predetermined value, this is determined that operation is performed using the guidance device 20 to observe the inside of the subject while the capsule endoscope 100 is held in a stationary state. In this case, the strength of the capsule detection magnetic field is increased, and the accuracy of detection of the position and the posture of the capsule endoscope 100 by the guidance device 20 is improved. Accordingly, the guidance device 20 can generate the guiding-magnetic field MF, in proximity to the capsule endoscope 100, for reliably holding the capsule endoscope 100 at the user desired position. It should be noted that when the power supply provided to the detection magnetic field generation unit 126 is already increased, it is sufficient to only maintain the level of the power supply in step S114. Thereafter, the operation of the capsule endoscope 100 returns back to the main routine.

### (Modification 1-1-3)

Subsequently, Modification 1-1-3 of Embodiment 1-1 will be explained.

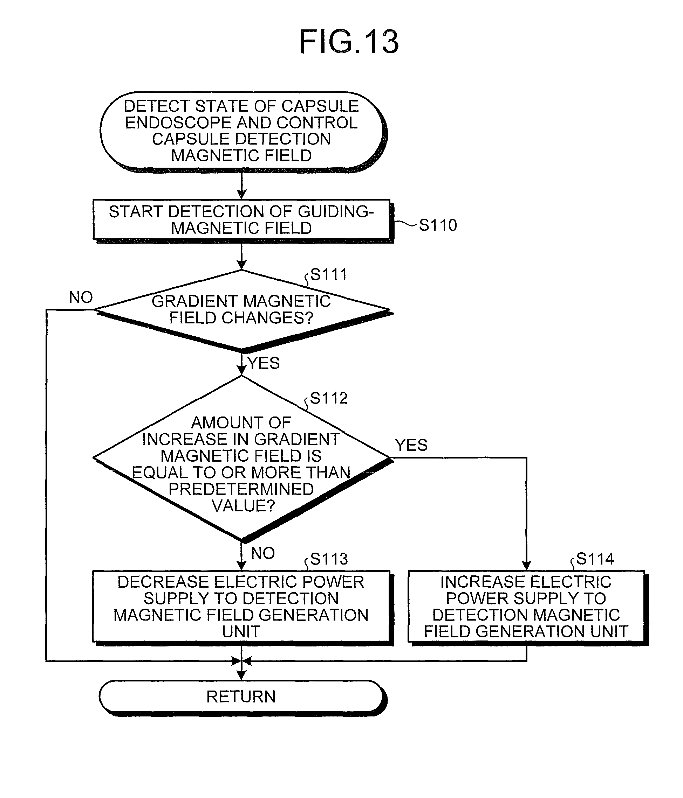

FIG. 13 is a flowchart illustrating control operation of state detection and capsule detection magnetic field of the capsule endoscope according to Modification 1-1-3 (step S14). When change of the gradient magnetic field is detected as the state of the capsule endoscope 100, a control which is opposite to Modification 1-1-2 may be performed.

More specifically, when the amount of increase in the gradient magnetic field is determined to be equal to or more than the predetermined value as a result of the determination in step S112 (step S112: Yes), the detection magnetic field controller 127 increases the power supply provided to the detection magnetic field generation unit 126 in step S114. Accordingly, the strength of the capsule detection magnetic field is increased.

In this case, when the amount of increase in the gradient magnetic field is larger than the predetermined value, this is determined that the guidance device 20 is performing control for forcibly moving the capsule endoscope 100. In this case, by increasing the strength of the capsule detection magnetic field, the accuracy of detection of the position and the posture of the capsule endoscope 100 by the guidance device 20 can be improved. As a result, the guiding-magnetic field MF for guiding the position and the posture of the capsule endoscope 100 (gradient magnetic field) can be generated, in proximity to the capsule endoscope 100, in a more reliable and efficient manner. It should be noted that when the power supply provided to the detection magnetic field generation unit 126 is already increased, it is sufficient to only maintain the level of the power supply in step S114.

On the other hand, when the amount of increase in the gradient magnetic field is less than the predetermined value as a result of the determination in step S112 (step S112: No), the detection magnetic field controller 127 decreases the power supply provided to the detection magnetic field generation unit 126 in step S113. Accordingly, the strength of the detection magnetic field is reduced.

In this case, when the amount of increase in the gradient magnetic field is less than the predetermined value, i.e., when normal magnetic gradient is applied to the capsule endoscope 100, this is determined that the guidance device 20 is successfully guiding the capsule endoscope 100 in accordance with the user's intention. In this case, it is not necessary for the guidance device 20 to detect the position and the posture of the capsule endoscope 100 with so much high degree of accuracy, and therefore, the generation strength of the detection magnetic field is decreased, and the electric power that is consumed by the capsule endoscope 100 can be suppressed.

### (Modification 1-1-4)

Subsequently, Modification 1-1-4 of Embodiment 1-1 will be explained.

When the change of the gradient magnetic field is detected in control operation of the state detection and capsule detection magnetic field of the capsule endoscope (step S14), the amount of increase in the gradient magnetic field may be determined in a stepwise manner, and the power supply provided to the detection magnetic field generation unit 126 may be controlled.

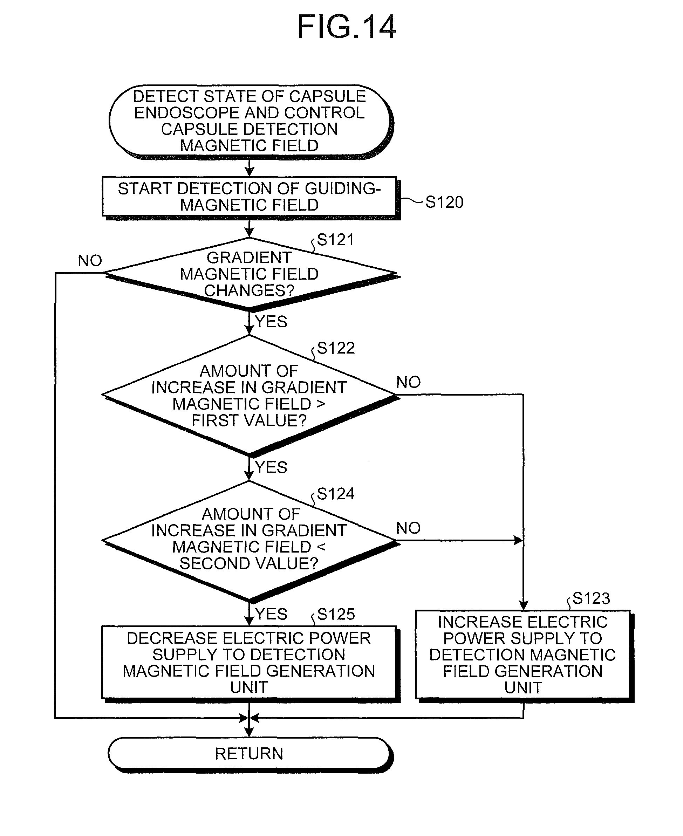

FIG. 14 is a flowchart illustrating operation of the capsule endoscope 100 in step S14 according to Modification 1-1-4.

More specifically, first, in step S120, the guiding-magnetic field detector 125 starts the detection of the guiding-magnetic field MF (gradient magnetic field).

In step S121 subsequent thereto, the detection magnetic field controller 127 determines whether the gradient magnetic field is changed or not, from the output signal of the guiding-magnetic field detector 125. When the gradient magnetic field is changed (step S121: Yes), the operation of the capsule endoscope 100 proceeds to step S122. On the other hand, when the gradient magnetic field is not changed (step S121: No), the operation of the capsule endoscope 100 returns back to the main routine.

In step S122, the detection magnetic field controller 127 determines whether the amount of increase in the gradient magnetic field is more than a first value determined in advance. When the amount of increase in the gradient magnetic field is equal to or less than the first value (step S122: No), the detection magnetic field controller 127 increases the power supply provided to the detection magnetic field generation unit 126 (step S123). Accordingly, the strength of the capsule detection magnetic field is increased.

At this occasion, in this case, this is determined that at the guidance device 20, operation is performed to observe the inside of the subject while the capsule endoscope 100 is held in a stationary state. For this reason, by increasing the strength of the detection magnetic field, the accuracy of detection of the position and the posture of the capsule endoscope 100 by the guidance device 20 is improved. Therefore, the guidance device 20 can generate the guiding-magnetic field MF for reliably holding the capsule endoscope 100 at the user desired position in a stationary manner. Thereafter, the operation of the capsule endoscope 100 returns back to the main routine.

On the other hand, the amount of increase in the gradient magnetic field is more than the first value (step S122: Yes), the detection magnetic field controller 127 determines whether the amount of increase in the gradient magnetic field is less than a second value determined in advance (first value < second value) (step S124).

When the amount of increase in the gradient magnetic field is equal to or more than the second value (step S124: No), the detection magnetic field controller 127 increases the power supply provided to the detection magnetic field generation unit 126 (step S123). Accordingly, the strength of the capsule detection magnetic field is increased.

At this occasion, in this case, this is determined that at the guidance device 20, operation is performed to forcibly move the capsule endoscope 100. For this reason, by increasing the strength of the detection magnetic field, the accuracy of detection of the position and the posture of the capsule endoscope 100 by the guidance device 20 is improved. Therefore, the guiding-magnetic field MF for guiding the position and the posture of the capsule endoscope 100 (gradient magnetic field) can be generated, in proximity to the capsule endoscope 100, in a more reliable and efficient manner. When the power supply provided to the detection magnetic field generation unit 126 is already increased, it is sufficient to only maintain the level of the power supply in step S123.

When the amount of increase in the gradient magnetic field is less than the second value (step S124: Yes), and more specifically, when the amount of increase in the gradient magnetic field is between the first value and the second value, the detection magnetic field controller 127 decreases the power supply provided to the detection magnetic field generation unit 126 (step S125). Accordingly, the strength of the capsule detection magnetic field is decreased. This is because, the guidance device 20 can guide the capsule endoscope 100 in accordance with the user's intention, and this is determined that it is not necessary for the guidance device 20 to detect the position and the posture of the capsule endoscope 100 with so much high degree of accuracy. In this case, the generation strength of the detection magnetic field is decreased, and the electric power that is consumed by the capsule endoscope 100 can be suppressed. Thereafter, the operation of the capsule endoscope 100 returns back to the main routine.

It should be noted that, in Modification 1-1-4, the amount of increase in the gradient magnetic field is determined based on the first and second values, and in accordance with the determination result, the increase and the decrease in the power supply provided to the detection magnetic field generation unit 126 is controlled, but the reference value used for determining the amount of increase in the gradient magnetic field may be set in more details. In this case, in accordance with the setting of the reference value, the degree of the increase or the decrease in the power supply provided to the detection magnetic field generation unit 126 may be determined in a stepwise manner. Alternatively, in accordance with the amount of increase in the gradient magnetic field, the power supply provided to the detection magnetic field generation unit 126 may be increased or decreased in a gradient manner.

### (Modification 1-1-5)

Subsequently, Modification 1-1-5 of Embodiment 1-1 will be explained.

As illustrated in FIG. 2, when the guiding-magnetic field detector 125 is constituted by a three-axis magnetic field sensor, two three-axis magnetic field sensors may be arranged in directions in which the capsule endoscope 100 is to be guided with strong force. For example, when the two three-axis magnetic field sensors are arranged in parallel to the longitudinal axis La, the three-axis magnetic field sensors can detect the guiding-magnetic field MF for guiding the capsule endoscope 100 in the direction of the longitudinal axis La. In this case, when guiding operation is performed in the direction of the longitudinal axis La, determination is made as to whether to increase/decrease (or turn ON/OFF) the power supply provided to the detection magnetic field generation unit 126.

### (Modification 1-1-6)

Subsequently, Modification 1-1-6 of Embodiment 1-1 will be explained.

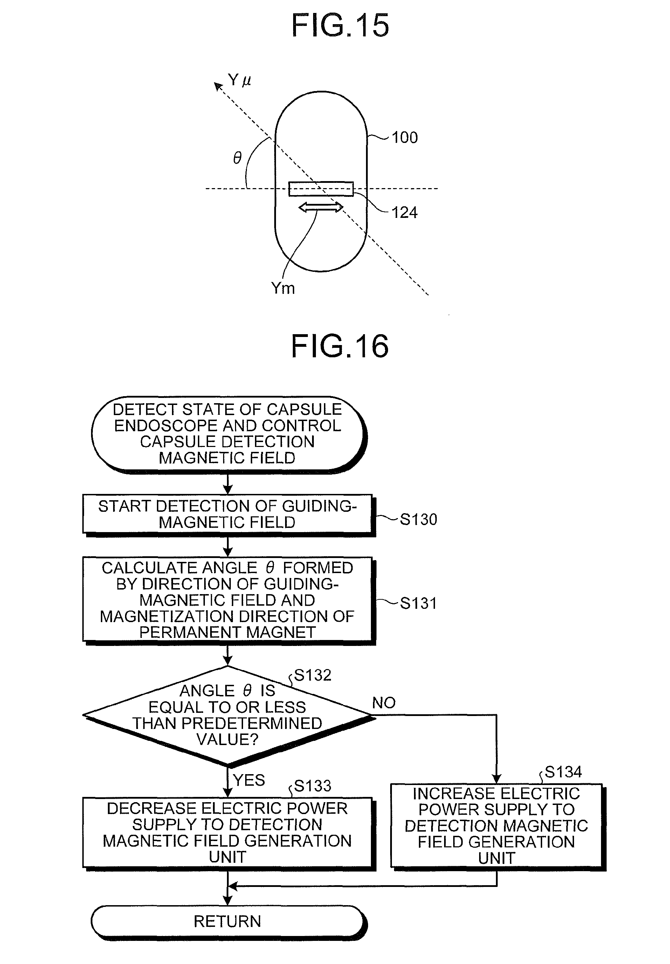

In control operation of the state detection and capsule detection magnetic field of the capsule endoscope (step S14), the power supply provided to the detection magnetic field generation unit 126 may be controlled based on the direction of the guiding-magnetic field MF for the capsule endoscope 100. For example, as illustrated in FIG. 15, the power supply is controlled based on the size of angle θ formed by the direction Yµ of the guiding-magnetic field MF and the magnetization direction Ym of the permanent magnet 124. In this case, the guiding-magnetic field detector 125 may be constituted by four three-axis magnetic field sensors (see FIG. 11) in the three-axis directions.

FIG. 16 is a flowchart illustrating operation of the capsule endoscope 100 in step S14 according to Modification 1-1-6.

First, in step S130, the guiding-magnetic field detector 125 starts the detection of the guiding-magnetic field MF.

In step S131 subsequent thereto, the detection magnetic field controller 127 acquires the direction Yµ of the guiding-magnetic field MF from the output signal of the guiding-magnetic field detector 125, and calculates the angle θ formed by the direction Yµ and the magnetization direction Ym of the permanent magnet 124.

When the calculated angle θ is equal to or less than a predetermined threshold value (predetermined value) (step S132: Yes), the detection magnetic field controller 127 decreases the power supply provided to the detection magnetic field generation unit 126 (step S133). Accordingly, the strength of the capsule detection magnetic field is decreased. In this case, when the angle θ is equal to or less than the predetermined value, the capsule endoscope 100 can be guided in accordance with guiding operation of the guidance device 20, and therefore, this is determined that it is not necessary for the guidance device 20 to detect the position and the posture of the capsule endoscope 100 with a high degree of accuracy. In this case, by decreasing the strength of the capsule detection magnetic field, the electric power that is consumed by the capsule endoscope 100 can be suppressed. Thereafter, the operation of the capsule endoscope 100 returns back to the main routine.

On the other hand, when the calculated angle θ is more than the predetermined value (step S132: No), the detection magnetic field controller 127 increases the power supply provided to the detection magnetic field generation unit 126 (step S134). Accordingly, the strength of the capsule detection magnetic field is increased. In this case, when the angle θ is more than the predetermined value, it is necessary to increase the strength of the guiding-magnetic field MF so that the magnetization direction Ym of the permanent magnet 124 becomes the same as the direction Yµ of the guiding-magnetic field MF. However, because of the increase in the strength of the guiding-magnetic field MF, the SN ratio of the detection signal of the capsule detection magnetic field may be deteriorated in the guidance device 20. Accordingly, by increasing the strength of the capsule detection magnetic field generated by the capsule endoscope 100, the deterioration of the SN ratio of the detection signal in the guidance device 20 may be suppressed. As a result, the guidance device 20 can accurately find the position and the posture of the capsule endoscope 100, and can generate the guiding-magnetic field MF, in proximity to the capsule endoscope 100, in a more accurate and efficient manner. It should be noted that when the power supply provided to the detection magnetic field generation unit 126 is already increased, it is sufficient to only maintain the level of the power supply in step S134.

### (Embodiment 1-2)

Subsequently, Embodiment 1-2 of the present invention will be explained.

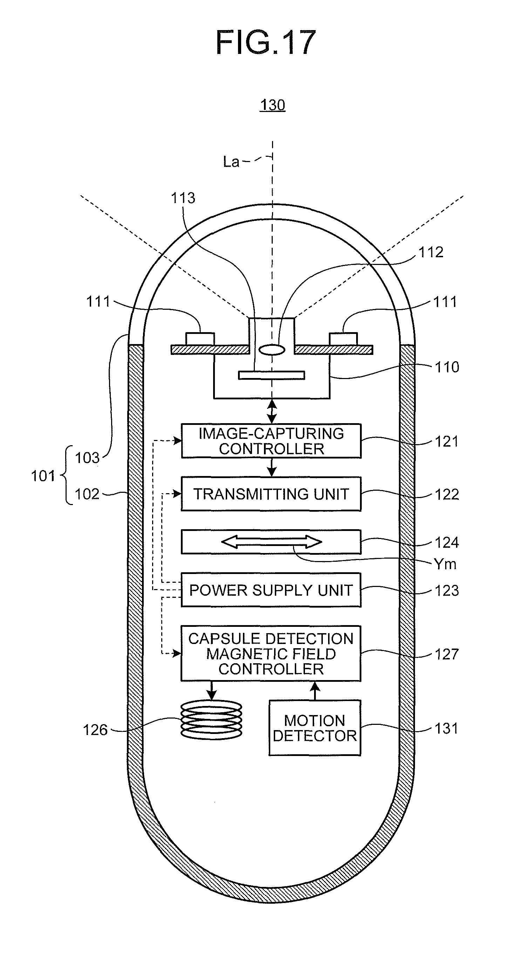

FIG. 17 is a schematic sectional view illustrating an example of an internal structure of a capsule endoscope according to Embodiment 1-2.

As illustrated in FIG. 17, a capsule endoscope 130 includes a motion detector 131 for detecting motion of the capsule endoscope 130 as a state detection unit for detecting the state of the capsule endoscope 130 instead of the guiding-magnetic field detector 125 as illustrated in FIG. 2. In Embodiment 1-2, the motion detector 131 is achieved with an acceleration sensor for detecting the acceleration of the capsule endoscope 130. It should be noted that the configuration of the capsule endoscope 130 except the motion detector 131 is the same as what is illustrated in FIG. 2.

Subsequently, the operation of the capsule endoscope 130 will be explained. The overall operation of the capsule endoscope 130 is the same as what is illustrated in FIG. 8, but the contents of the control operation of the state detection and capsule detection magnetic field of the capsule endoscope (step S14) are different from those of Embodiment 1-1.

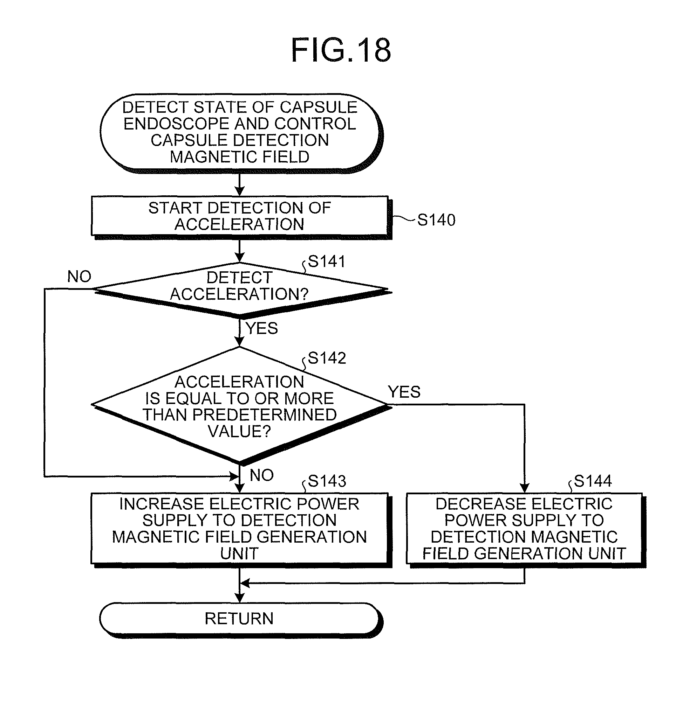

FIG. 18 is a flowchart illustrating operation of the capsule endoscope 130 in step S14.

First, in step S140, the motion detector 131 starts the detection of the acceleration of the capsule endoscope 130. In step S141, when the motion detector 131 detects the acceleration (step S141: Yes), the detection magnetic field controller 127 determines whether the detected acceleration is equal to or more than a predetermined threshold value (predetermined value) (step S142).

When the motion detector 131 does not detect the acceleration (step S141: No), or when the detected acceleration is less than a predetermined value (step S142: No), the detection magnetic field controller 127 increases the power supply provided to the detection magnetic field generation unit 126 (step S143), and increases the strength of the capsule detection magnetic field. Alternatively, when the power supply provided to the detection magnetic field generation unit 126 is in the OFF state, this is turned ON.

At this occasion, when the motion of the capsule endoscope 130 is small (or there is no motion), this is determined such that, at the guidance device 20, operation is performed to observe the inside of the subject while the capsule endoscope 130 is held in a stationary state. In this case, by increasing the strength of the capsule detection magnetic field, the accuracy of detection of the position and the posture of the capsule endoscope 130 by the guidance device 20 is improved. Accordingly, the guidance device 20 can be caused to generate the guiding-magnetic field MF for holding the capsule endoscope 130 at the user desired position in a stationary manner. It should be noted that when the power supply provided to the detection magnetic field generation unit 126 is already increased, it is sufficient to only maintain the level of the power supply in step S143. Thereafter, the operation of the capsule endoscope 130 returns back the main routine.

On the other hand, when the detected acceleration is equal to or more than a predetermined value (step S142: Yes), the detection magnetic field controller 127 decreases the power supply provided to the detection magnetic field generation unit 126 (step S144), the strength of the detection magnetic field is decreased. Alternatively, the power supply provided to the detection magnetic field generation unit 126 may be turned OFF. At this occasion, when the acceleration is equal to or more than the predetermined value, this is determined such that the capsule endoscope 130 is moving in accordance with the guiding operation given by the guidance device 20. In this case, the guidance device 20 does not need to accurately detect the position and the posture of the capsule endoscope 130, and therefore, by decreasing (or turning OFF) the strength of the detection magnetic field, the electric power that is consumed by the capsule endoscope 130 can be suppressed. Thereafter, the operation of the capsule endoscope 130 returns back to the main routine.

In the capsule endoscope 130, the adjustment of the power supply provided to the detection magnetic field generation unit 126 may be executed by not only adjusting the electric current flowing through the detection magnetic field generation unit 126 but also, like Modification 1-1-1, adjusting the interval according which the electric power is provided, or selecting a coil to which the electric power is provided from among multiple coils of which impedances are different.

### (Modification 1-2-1)

Subsequently, Modification 1-2-1 of Embodiment 1-2 will be explained.

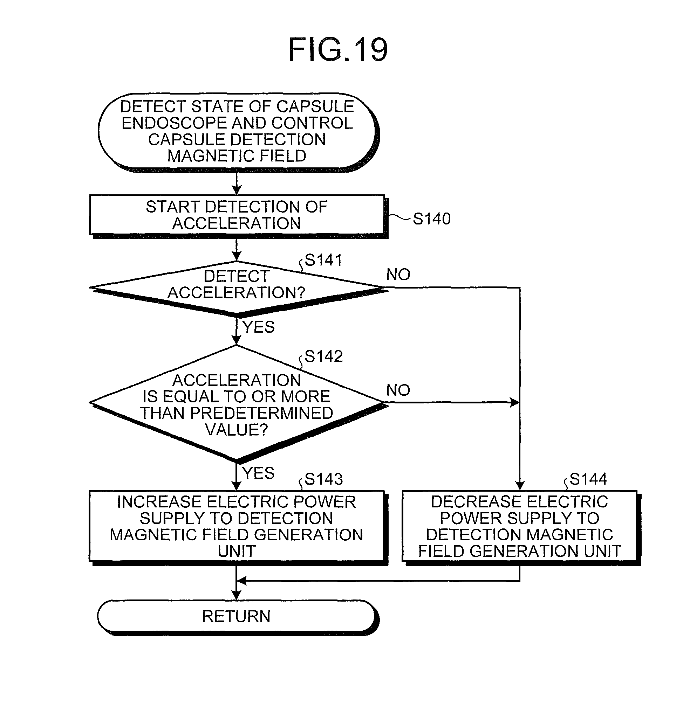

In the control operation of state detection and capsule detection magnetic field of the capsule endoscope (step S14), control which is opposite to Embodiment 1-2 may be performed. FIG. 19 is a flowchart illustrating operation of the capsule endoscope 130 in step S14 according to Modification 1-2-1.

More specifically, when the detected acceleration is equal to or more than the predetermined value as a result of the determination in step S142 (step S142: Yes), the detection magnetic field controller 127 increases the power supply provided to the detection magnetic field generation unit 126 (step S143), and the strength of the detection magnetic field is increased. On the other hand, when no acceleration is detected as a result of determination in steps S141 and S142 (step S141: No), or when the detected acceleration is less than the predetermined value (step S142: No), the detection magnetic field controller 127 decreases the power supply provided to the detection magnetic field generation unit 126 (step S144), and the strength of the detection magnetic field is reduced. As described above, only when the motion of the capsule endoscope 130 is great, the strength of the detection magnetic field may be increased to improve the accuracy of detection of the capsule endoscope 130 in the guidance device 20, and in the other cases, the strength of the detection magnetic field may be suppressed, whereby the electric power that is consumed by the capsule endoscope 130 may be suppressed. It should be noted that, when the power supply provided to the detection magnetic field generation unit 126 is already increased, it is sufficient to only maintain the level of the power supply in step S143.

### (Modification 1-2-2)

Subsequently, Modification 1-2-2 of Embodiment 1-2 will be explained.

In the control operation of state detection and capsule detection magnetic field of the capsule endoscope (step S14), when the strength of the detection magnetic field is changed based on the motion of the capsule endoscope 130, and the power supply provided to the detection magnetic field generation unit 126 may be controlled by determining the amount of motion in a stepwise manner.

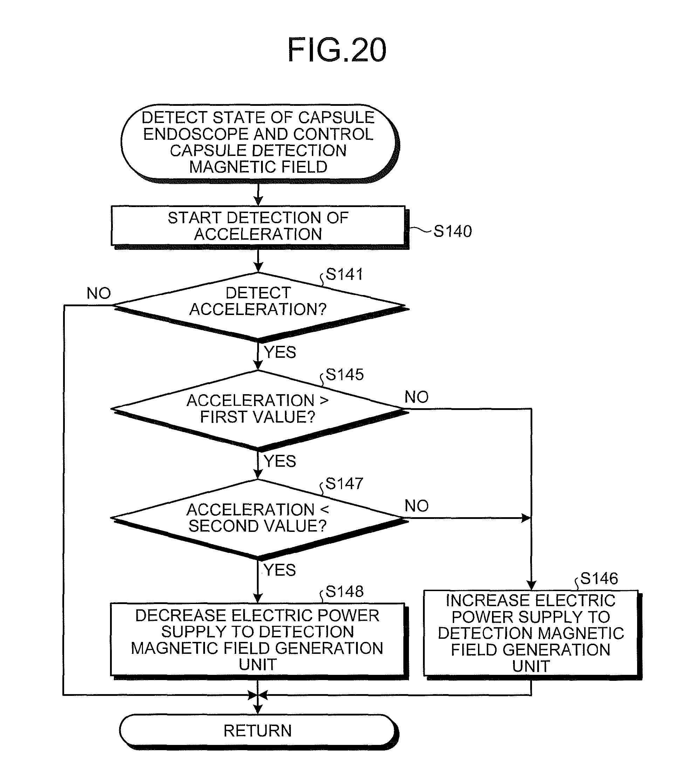

FIG. 20 is a flowchart illustrating operation of the capsule endoscope 130 in step S14 according to Modification 1-2-2.

First, in step S140, the motion detector 131 starts the detection of the acceleration of the capsule endoscope 130. When the motion detector 131 detects the acceleration in step S141 (step S141: Yes), the operation of the capsule endoscope 130 proceeds to step S145. On the other hand, when the motion detector 131 does not detect the acceleration (step S141: No), the operation of the capsule endoscope 130 returns back to the main routine.

In step S145, the detection magnetic field controller 127 determines whether the detected acceleration is more than a first value determined in advance. When the acceleration is equal to or less than the first value (step S145: No), the detection magnetic field controller 127 increases the power supply provided to the detection magnetic field generation unit 126 (step S146), the strength of the capsule detection magnetic field is increased. This is because it is determined that, at the guidance device 20, operation is performed to observe the inside of the subject while the capsule endoscope 130 is held in a stationary state, and therefore, the accuracy of detection of the position and the posture of the capsule endoscope 130 is to be improved in the guidance device 20. It should be noted that the power supply provided to the detection magnetic field generation unit 126 is already increased, it is sufficient to only maintain the level of the power supply in step S146. Thereafter, the operation of the capsule endoscope 130 returns back to the main routine.

On the other hand, when the acceleration is more than the first value (step S145: Yes), the detection magnetic field controller 127 determines whether the acceleration is less than a second value determined in advance (first value < second value) (step S147).

When the acceleration is equal to or more than the second value (step S147: No), the detection magnetic field controller 127 increases the power supply provided to the detection magnetic field generation unit 126 (step S146), and the strength of the capsule detection magnetic field is increased. This is because it is determined that, at the guidance device 20, operation is performed to forcibly move the capsule endoscope 130, and therefore, the accuracy of detection of the position and the posture of the capsule endoscope 130 is to be improved in the guidance device 20. It should be noted that when the power supply provided to the detection magnetic field generation unit 126 is already increased, it is sufficient to only maintain the level of the power supply in step S146.

When the acceleration is less than the second value (step S147: Yes), more specifically, when the acceleration is between the first value and the second value, the detection magnetic field controller 127 decreases the power supply provided to the detection magnetic field generation unit 126 (step S148). This is because it is determined that, at the guidance device 20, the capsule endoscope 130 can be guided in accordance with the user's intention, and it is not necessary for the guidance device 20 to detect the position and the posture of the capsule endoscope 130 with so much high degree of accuracy. In this case, by reducing the generation strength of the detection magnetic field, the electric power that is consumed by the capsule endoscope 130 can be suppressed. Thereafter, the operation of the capsule endoscope 130 returns back to the main routine.

### (Modification 1-2-3)

Subsequently, Modification 1-2-3 of Embodiment 1-2 will be explained.

In the control operation of state detection and capsule detection magnetic field of the capsule endoscope (step S14), in a case where the strength of the detection magnetic field is to be changed based on the motion of the capsule endoscope 130, and the amount of motion is less than a predetermined value, then the power supply provided to the detection magnetic field generation unit 126 may be increased. In this case, when the amount of motion of the capsule endoscope 130 is small, this means that the capsule endoscope 130 is determined to have been restrained by residual in the pleats of the intestine wall and the lumens. In such case, it is necessary for the guidance device 20 to increase the strength of the guiding-magnetic field MF, and apply it to the capsule endoscope 130, so that the capsule endoscope 130 is moved. On the other hand, when the strength of the guiding-magnetic field MF is increased, the SN ratio of the detection signal of the detection magnetic field at the guidance device 20 is deteriorated, and the accuracy of detection of the capsule endoscope 130 may be reduced.

Accordingly, when the amount of motion of the capsule endoscope 130 is small, the power supply provided to the detection magnetic field generation unit 126 is increased, and the strength of the detection magnetic field is increased, so that the deterioration of the SN ratio of the detection signal of the detection magnetic field is suppressed, and the capsule endoscope 130 can reliably move in accordance with the guiding operation of the guidance device 20. When the motion of the capsule endoscope 130 is equal to or more than the predetermined value, the power supply provided to the detection magnetic field generation unit 126 may be returned back to the original value (low level value).

### (Modification 1-2-4)

Subsequently, Modification 1-2-4 of Embodiment 1-2 will be explained.

In Embodiment 1-2 explained above, the acceleration sensor is used to detect the acceleration of the capsule endoscope 130, but when the physical quantity corresponding to the acceleration can be detected, anything other than the acceleration sensor may be used. Other than the acceleration, the motion detector 131 may be achieved with any configuration as long as the motion of the capsule endoscope 130 can be detected. It should be noted that the control method of the power supply is the same as what has been explained in Embodiment 1-2 and Modification 1-2-1 thereof.

For example, a gravity sensor may be provided as the motion detector 131. In this case, the detection magnetic field controller 127 can detect the speed and presence/absence of the posture change of the capsule endoscope 130 from the output change of the gravity sensor.

An angular speed sensor (gyroscope) may be provided as the motion detector 131. In this case, the detection magnetic field controller 127 can detect the speed and presence/absence of the posture change of the capsule endoscope 130 from the output change of the angular speed sensor.

A water pressure sensor may be provided as the motion detector 131. In this case, the detection magnetic field controller 127 can calculate the speed and presence/absence of the motion of the capsule endoscope 130 in the vertical direction, from the change of the detection value of the water pressure sensor (water pressure).

An ultrasonic wave sensor can be provided as the motion detector 131 to generate an ultrasonic wave and detect an ultrasonic wave reflected by a subject (reflection wave). In this case, the detection magnetic field controller 127 can calculate the moving direction and the moving speed of the capsule endoscope 130 from the change of the phase and the change of the frequency of the detected ultrasonic wave.

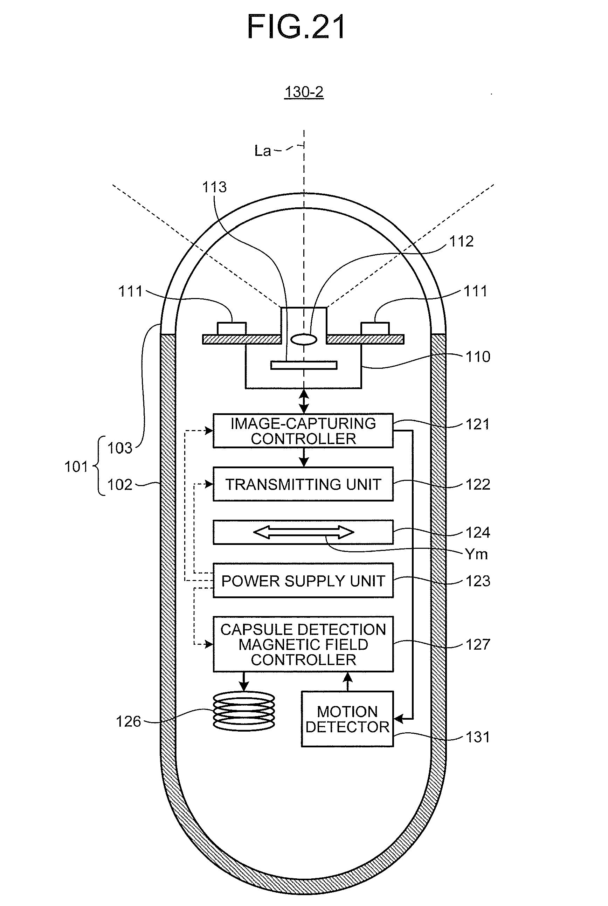

As illustrated by a capsule endoscope 130-2 of FIG. 21, image data which are captured by the image-capturing unit 110 and on which predetermined signal processing is performed by the image-capturing controller 121 are input into the motion detector 131, and based on the image information successively acquired by the image-capturing unit 110, the motion of the capsule endoscope 130-2 may be detected. In this case, the motion detector 131 calculates difference between image data corresponding to two in-vivo images captured in order (for example, the number of pixels in two-value image performed threshold value processing on a difference image). When this difference is equal to or more than a predetermined amount, the detection magnetic field controller 127 controls the power supply provided to the detection magnetic field generation unit 126 by determining that the capsule endoscope 130-2 has moved.

The light adjustment information associated with image data of each frame is input into the motion detector 131, and based on the light adjustment information when each image is captured, the motion of the capsule endoscope 130-2 may be detected. In this case, the motion detector 131 calculates the change of the light adjustment information which is successively input (for example, difference of light emission strength). When this change is equal to or more than a predetermined amount, the detection magnetic field controller 127 determines that the capsule endoscope 130-2 has moved, and controls the power supply provided to the detection magnetic field generation unit 126.

### (Modification 1-2-5)

Subsequently, Modification 1-2-5 of Embodiment 1-2 will be explained.

The motion of the capsule endoscope may be detected using gradient field formed in the space including the capsule endoscope.

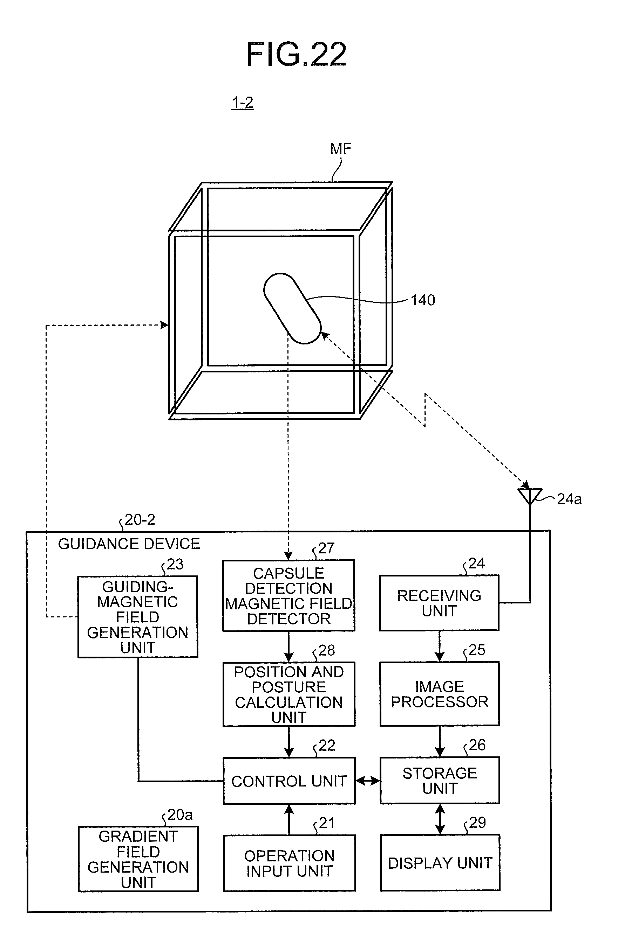

FIG. 22 is a block diagram illustrating a configuration of a medical system according to Modification 1-2-5. As illustrated in FIG. 22, a medical system 1-2 according to Modification 1-2-5 includes a guidance device 20-2 further including a gradient field generation unit 20a for the guidance device 20 as illustrated in FIG. 1 and a capsule endoscope 140.

The gradient field generation unit 20a generates, from the outside of the subject, the gradient field that does not change over time, in the space including the capsule endoscope 140. The gradient field generation unit 20a is achieved with, for example, an oscillator generating an ultrasonic wave by applying voltage, a coil and a direct current power supply for generating for generating a static magnetic field, and a coil and an alternate current power supply for generating an alternate current magnetic field, and an antenna for forming electric field. When an alternate current magnetic field is generated as the gradient field, the capsule detection magnetic field generated by the detection magnetic field generation unit 126 and the frequency may be caused to be different.

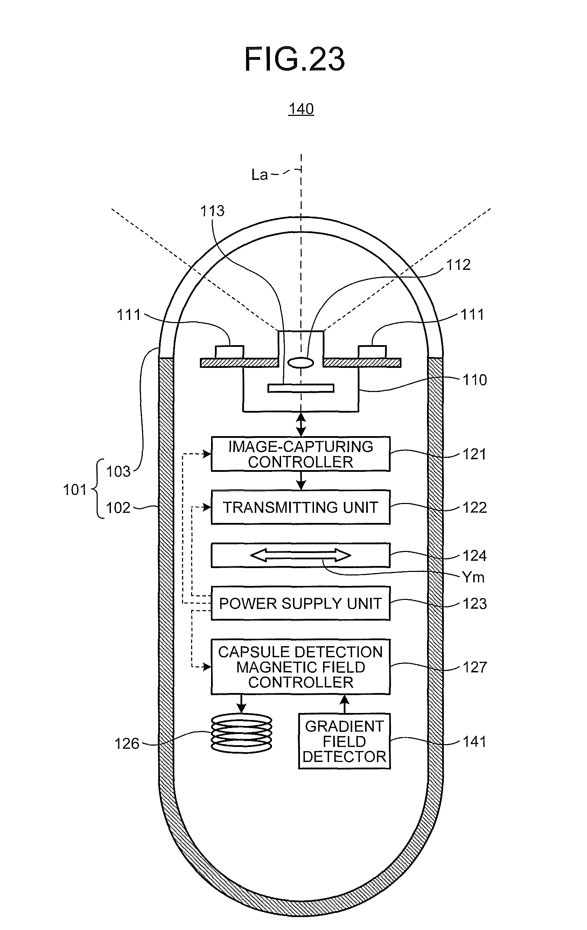

FIG. 23 is a schematic sectional view illustrating an example of an internal structure of the capsule endoscope 140. The capsule endoscope 140 includes a gradient field detector 141 as a motion detector for detecting the motion as the state of itself. More specifically, the gradient field detector 141 corresponds to the configuration of the gradient field generation unit 20a, and is constituted by, e.g., an oscillator for generating an electric signal upon receiving an ultrasonic wave, a coil for generating an electric signal upon detecting a magnetic field, and an antenna for outputting a detection signal upon detecting an electric field.

The detection magnetic field controller 127 detects the motion of the capsule endoscope 140 (the moving direction and the moving speed) based on the signal which is output from the gradient field detector 141. Then, in accordance with this detection result, the power supply provided to the detection magnetic field generation unit 126 is controlled.

### (Embodiment 1-3)

Subsequently, Embodiment 1-3 of the present invention will be explained.

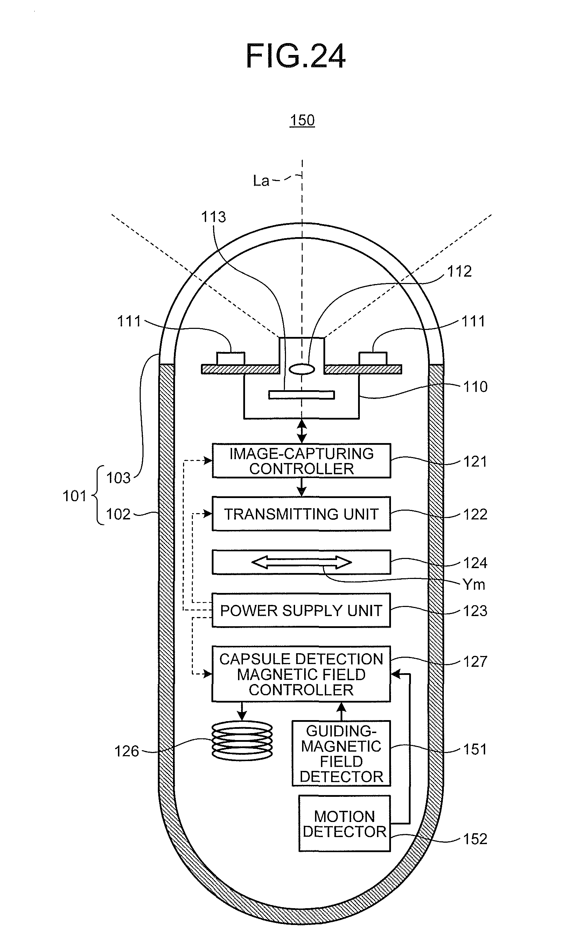

FIG. 24 is a schematic sectional view illustrating an example of an internal structure of a capsule endoscope according to Embodiment 1-3. As illustrated in FIG. 24, a capsule endoscope 150 includes a guiding-magnetic field detector 151 and a motion detector 152 serving as a state detection unit for detecting the state of the capsule endoscope 150. The detection magnetic field controller 127 controls the power supply provided to the detection magnetic field generation unit 126 in accordance with the detection result of the guiding-magnetic field detector 151 and the motion detector 152.

The configuration and the operation of the guiding-magnetic field detector 151 are the same as the configuration and the operation of the guiding-magnetic field detector 125 that has been explained in Embodiment 1-1 and the modification thereof. More specifically, the guiding-magnetic field detector 151 is achieved with four three-axis magnetic field sensors capable of detecting the magnetic field in the three-axis direction. The configuration and the operation of the motion detector 152 are the same as the configuration and the operation of the motion detector 131 that has been explained in Embodiment 1-2 and the modification thereof. More specifically, the motion detector 152 is achieved with, e.g., an acceleration sensor, a water pressure sensor, and an ultrasonic wave sensor capable of detecting the direction of the motion of the capsule endoscope 150, or a calculation unit for detecting difference of image information or light adjustment information.

Subsequently, the operation of the capsule endoscope 150 will be explained. The overall operation of the capsule endoscope 150 is the same as what has been illustrated in FIG. 8, and the contents of the control operation of the state detection and the capsule detection magnetic field of the capsule endoscope (step S14) are different from those of Embodiment 1-1.

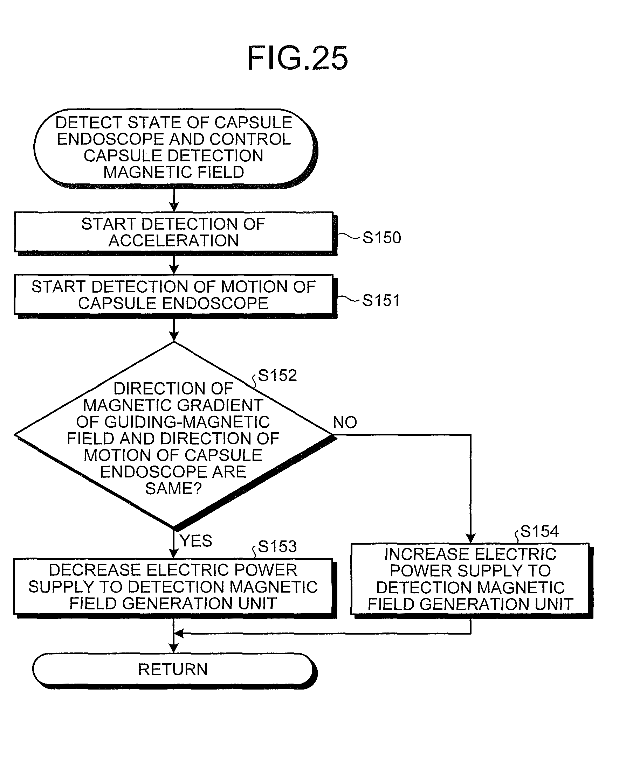

FIG. 25 is a flowchart illustrating operation of the capsule endoscope 150 in step S14.

First, in step S150, the guiding-magnetic field detector 151 starts detection of the guiding-magnetic field MF (for example, the direction and the strength of the magnetic gradient). In step S151, the motion detector 152 starts detection of the motion of the capsule endoscope 150 (for example, the direction and the acceleration of the motion).

In step S152 subsequent thereto, the detection magnetic field controller 127 determines whether the guiding operation calculated from the detected guiding-magnetic field MF and the motion of the capsule endoscope 150 are the same or not.

When the guiding-magnetic field MF and the direction of the motion of the capsule endoscope 150 are the same, and more specifically, when the angle formed by the direction of the magnetic gradient detected by the guiding-magnetic field detector 151 and the direction of the acceleration detected by the motion detector 152 is within a predetermined angle, and the difference between the acceleration of the capsule endoscope 150 calculated from the strength of the guiding-magnetic field MF detected by the guiding-magnetic field detector 151 and the acceleration detected by the motion detector 152 is within a predetermined value (step S152: Yes), then the detection magnetic field controller 127 decreases the power supply provided to the detection magnetic field generation unit 126 (step S153), and the strength of the capsule detection magnetic field is decreased. Alternatively, the power supply may be turned off. This is because the capsule endoscope 150 is determined to be moving in accordance with guiding operation of the guidance device 20, and therefore, it is not necessary for the guidance device 20 to detect the capsule endoscope 150 with a high degree of accuracy. Thereafter, operation of the capsule endoscope 150 returns back to the main routine.

On the other hand, when the magnetic gradient and the motion of the capsule endoscope 150 are not the same, more specifically, when the angle formed by the directions is more than the predetermined angle, or when the difference of the acceleration is more than the predetermined value (step S152: No), the detection magnetic field controller 127 increases the power supply provided to the detection magnetic field generation unit 126 (step S154), and the strength of the capsule detection magnetic field is increased. Alternatively, when the power supply is in the OFF state, this may be turned on. This is because the capsule endoscope 150 is determined not to be moving in accordance with the guiding operation of the guidance device 20, and therefore, it is not necessary for the guidance device 20 to detect the capsule endoscope 150 with a high degree of accuracy. When the power supply provided to the detection magnetic field generation unit 126 is already increased, it is sufficient to only maintain the level of the power supply in step S154. Thereafter, operation of the capsule endoscope 150 returns back to the main routine.

As described above, according to Embodiment 1-3, when the capsule endoscope 150 moves in accordance with the guiding operation of the guidance device 20, the strength of the detection magnetic field is decreased, and therefore, the electric power that is consumed by the capsule endoscope 150 can be suppressed.

### (Modification 1-3-1)

Subsequently, Modification 1-3-1 of Embodiment 1-3 will be explained.

In the control operation of state detection and capsule detection magnetic field of the capsule endoscope (step S14), when the strength of the detection magnetic field is controlled based on the guiding-magnetic field MF that is applied to the capsule endoscope 150, the control may be performed based on the direction Yµ of the guiding-magnetic field MF and the posture of the capsule endoscope 150. In this case, the motion detector 152 may be achieved with a gravity sensor or an angular speed sensor capable of detecting the change of the posture of the capsule endoscope 150 (the gradient angle of the longitudinal axis La of the capsule endoscope 150 with respect to the vertical axis).

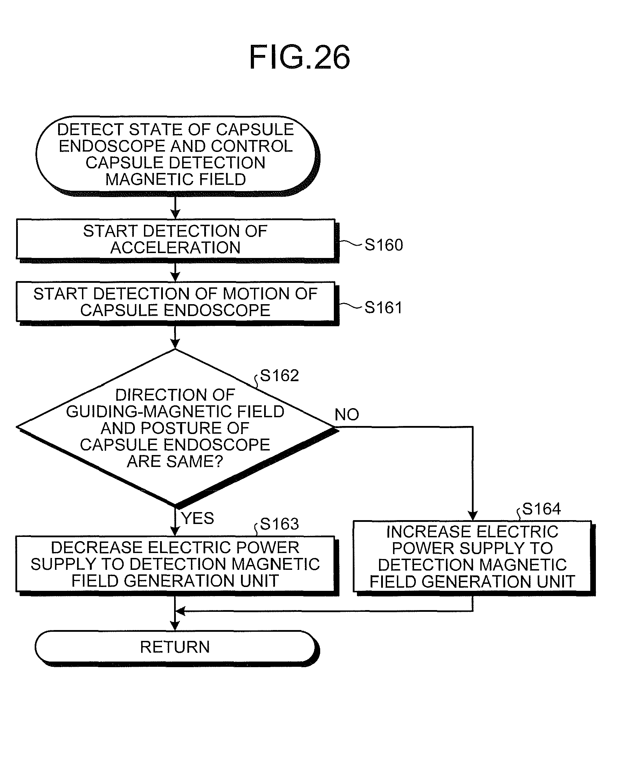

FIG. 26 is a flowchart illustrating operation of the capsule endoscope 150 in step S14 according to Modification 1-3-1.

First, in step S160, the guiding-magnetic field detector 151 starts the detection of the guiding-magnetic field MF. In step S161, the motion detector 152 starts the detection of the change of the posture of the capsule endoscope 150 (gradient angle).

In step S162, the detection magnetic field controller 127 determines whether the direction of the guiding-magnetic field MF and the posture of the capsule endoscope 150 are the same.

When the direction Yµ of the guiding-magnetic field MF and the posture of the capsule endoscope 150 are the same (step S162: Yes), the detection magnetic field controller 127 decreases the power supply provided to the detection magnetic field generation unit 126 (step S163), the strength of the detection magnetic field is decreased.

On the other hand, when the direction Yµ of the guiding-magnetic field MF and the posture of the capsule endoscope 150 are not the same (step S162: No), the detection magnetic field controller 127 increases the power supply provided to the detection magnetic field generation unit 126 (step S164), and the strength of the detection magnetic field is increased. It should be noted that when the power supply provided to the detection magnetic field generation unit 126 is already increased, it is sufficient to only maintain the level of the power supply in step S164.

As described above, according to Modification 1-3-1, when the capsule endoscope 150 is operating in accordance with the guiding operation by the guidance device 20 (change of the guiding-magnetic field MF), the strength of the capsule detection magnetic field is decreased, and therefore, the electric power that is consumed by the capsule endoscope 150 can be suppressed.

### (Embodiment 1-4)

Subsequently, Embodiment 1-4 of the present invention will be explained.

A capsule endoscope according to Embodiment 1-4 calculates, with the capsule endoscope 130 as illustrated in FIG. 17, the motion of the capsule endoscope 130 from the strength of the guiding-magnetic field MF that is applied to the capsule endoscope 130. In this case, the motion detector 131 is constituted by four three-axis magnetic field sensors (see FIG. 11) arranged in three-axis (X axis, Y axis, Z axis) directions.

The operation of the capsule endoscope 130 according to Embodiment 1-4 will be explained. The overall operation of the capsule endoscope 130 is the same as what has been described in FIG. 8, and the contents of the control operation of state detection and capsule detection magnetic field of the capsule endoscope (step S14) are different from those of Embodiment 1-1.

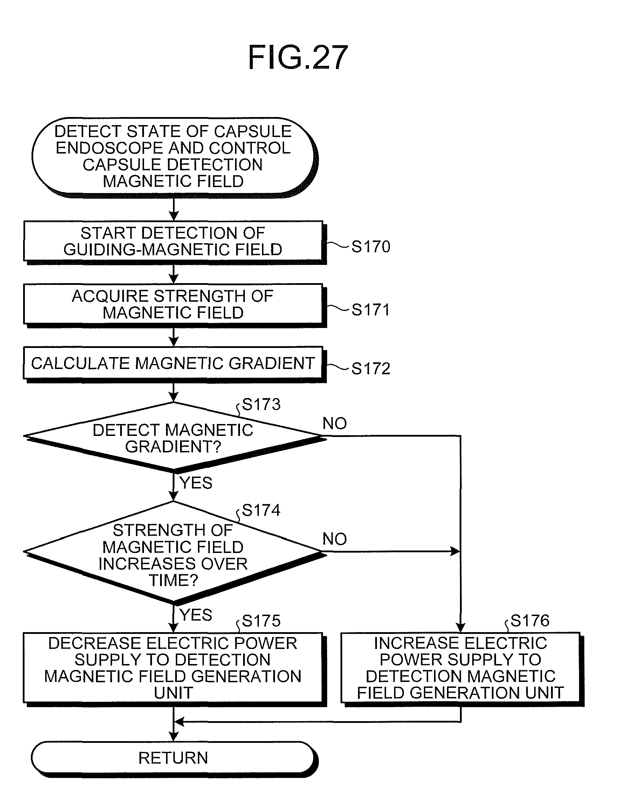

FIG. 27 is a flowchart illustrating operation of the capsule endoscope 130 in step S14.

First, in step S170, the motion detector 131 starts detection of the guiding-magnetic field MF (strength).

In step S171 subsequent thereto, the detection magnetic field controller 127 acquires the strength of the guiding-magnetic field MF that is applied to the capsule endoscope 130, from the output signal of the guiding-magnetic field detector 125. This strength may be at least one of detection values of the four three-axis magnetic field sensors, or may be an average value of the detection values of the four three-axis magnetic field sensors.

In step S172, the detection magnetic field controller 127 calculates the magnetic gradient in each axis (X axis, Y axis, Z axis) from the magnetic field strength acquired by each three-axis magnetic sensor of the guiding-magnetic field detector 125.

In step S173, the detection magnetic field controller 127 determines whether the magnetic gradient is detected or not from the calculation result in step S172.

When the magnetic gradient is detected (step S173: Yes), the detection magnetic field controller 127 determines whether the strength of the magnetic field that is applied to the capsule endoscope 130 increases over time in step S174 subsequent thereto.

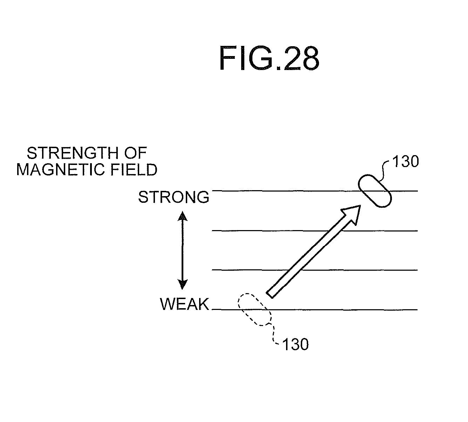

When the strength of the magnetic field increases over time (step S174: Yes), the detection magnetic field controller 127 decreases the power supply provided to the detection magnetic field generation unit 126 (step S175). In this case, when the magnetic field that is applied to the capsule endoscope 130 is inclined, and the strength of the magnetic field is increasing, this means that, as illustrated in FIG. 28, the capsule endoscope 130 is moving along the gradient of the magnetic field from the place where the strength is low to the place where the strength is high. More specifically, this means that the capsule endoscope 130 is moving in accordance with the guiding operation of the guidance device 20. In this case, by reducing the generation strength of the capsule detection magnetic field, the electric power that is consumed by the capsule endoscope 130 can be suppressed. Thereafter, the operation of the capsule endoscope 130 returns back to the main routine.

On the other hand, when the magnetic gradient is not detected (step S173: No), the detection magnetic field controller 127 increases the power supply provided to the detection magnetic field generation unit 126 (step S176). In this case, when the magnetic gradient is not detected, it can be determined that, in the guidance device 20, control is performed to hold the capsule endoscope 130 in a stationary state. In this case, the power supply provided to the detection magnetic field generation unit 126 is increased, and the strength of the capsule detection magnetic field is increased, so that the accuracy of detection of the capsule endoscope 130 by the guidance device 20 is improved. Accordingly, the guiding operation performance for the capsule endoscope 130 by the guidance device 20 is improved. When the power supply provided to the detection magnetic field generation unit 126 is already increased, it is sufficient to only maintain the level of the power supply in step S176, and thereafter, the operation of the capsule endoscope 130 returns back the main routine.

When the strength of the magnetic field does not change, or when it decreases over time (step S174: No), the detection magnetic field controller 127 increases the power supply provided to the detection magnetic field generation unit 126 in step S176. This is because, although the guidance device 20 performs the guiding operation for moving the capsule endoscope 130, the capsule endoscope 130 is determined not to be moving in accordance with the operation. Even in this case, the strength of the capsule detection magnetic field is increased, and the accuracy of detection of the capsule endoscope 130 by the guidance device 20 is improved, so that the guiding operation performance for the capsule endoscope 130 is improved. Also in this case, when the power supply provided to the detection magnetic field generation unit 126 is already increased, it is sufficient to only maintain the level of the power supply in step S176, and thereafter, the operation of the capsule endoscope 130 returns back to the main routine.

### (Embodiment 2-1)

Subsequently, Embodiment 2-1 of the present invention will be explained.

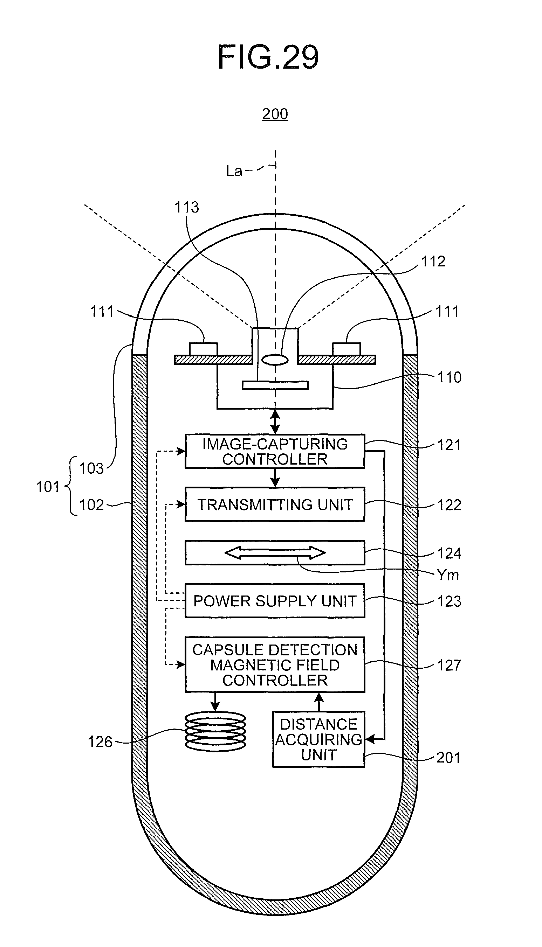

FIG. 29 is a schematic sectional view illustrating an example of an internal structure of a capsule endoscope provided in a medical system according to Embodiment 2-1. The configuration of the entire medical system according to Embodiment 2-1 is the same as what has been illustrated in FIG. 1. Embodiment 2-1 is characterized in that, based on the environment in which the capsule endoscope is placed, the strength of the detection magnetic field is controlled. In this case, the environment means the characteristics of the space around the capsule endoscope, and more specifically, it includes, e.g., geometrical characteristics such as the distance from an image-capturing target and the size of the ambient space, and physical and chemical characteristics such as liquid (mucus) viscosity, and pH existing therearound.

As illustrated in FIG. 29, a capsule endoscope 200 according to Embodiment 2-1 includes a distance acquiring unit 201 instead of the guiding-magnetic field detector 125 as illustrated in FIG. 2. The distance acquiring unit 201 is a form of a state detection unit for detecting the state of the capsule endoscope 200, and detects the environment in which the capsule endoscope 200 is placed. More specifically, the distance acquiring unit 201 detects the distance between the capsule endoscope 200 and an image-capturing target in the subject (the inner wall of an internal organ and the like) (which may also be hereinafter simply referred to as a distance from the image-capturing target).

The distance acquiring unit 201 retrieves image information (image data) captured by the image-capturing unit 110 in order and subjected to predetermined signal processing by the image-capturing controller 121, and acquires the distance from the image-capturing target from the image information. More specifically, for example, the distance acquiring unit 201 acquires predetermined color component in a pixel value of each pixel from image information (for example, red color component), and calculates the amount of attenuation of the color component (the strength of the image-captured signal for the emission strength), thus calculating the distance. In this case, a color component having a relatively long wavelength such a red color scatters less greatly in the subject, and therefore, the attenuation characteristics according to the distance from the image-capturing target are likely to be represented. Accordingly, in advance, a calculation expression or a table indicating correspondence relationship between the distance from the image-capturing target and the amount of attenuation is prepared, so that based on the color component, the distance from the image-capturing target can be acquired.

The detection magnetic field controller 127 controls the power supply provided to the detection magnetic field generation unit 126 based on the distance acquired as described above.

Subsequently, the operation of the capsule endoscope 200 will be explained. The operation of the entire capsule endoscope 200 is the same as what has been illustrated in FIG. 8, but the contents of the control operation of state detection and capsule detection magnetic field of the capsule endoscope (step S14) are different from those of Embodiment 1-1.

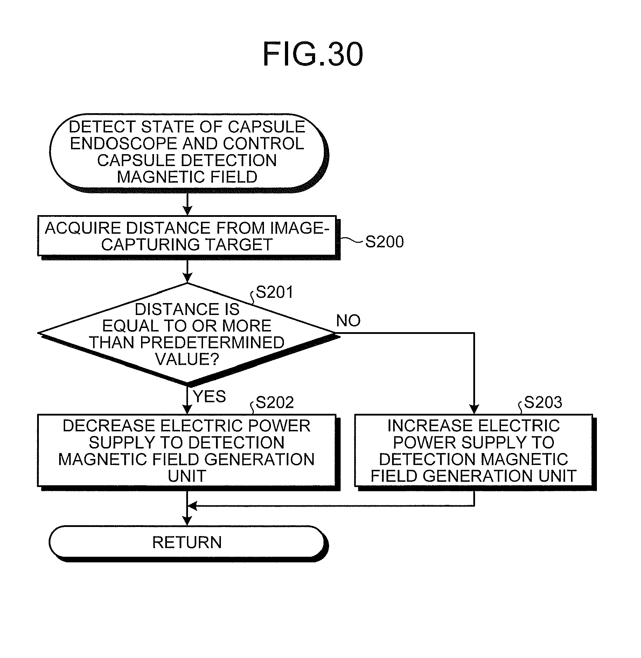

FIG. 30 is a flowchart illustrating operation of the capsule endoscope 200 in step S14.

First, in step S200, the distance acquiring unit 201 acquires image information from the image-capturing controller 121 in order and applies predetermined image processing, thus acquiring the distance from the image-capturing target.

In step S201 subsequent thereto, the detection magnetic field controller 127 determines whether the distance acquired by the distance acquiring unit 201 is equal to or more than a predetermined threshold value (predetermined value).

In step S201, when the distance is equal to or more than the predetermined value (step S201: Yes), the detection magnetic field controller 127 decreases the power supply provided to the detection magnetic field generation unit 126 (step S202), and the strength of the capsule detection magnetic field is decreased. In this case, when the distance from the image-capturing target is equal to or more than the predetermined value, more specifically, when the capsule endoscope 200 is far from the image-capturing target, then the guidance device 20 roughly performs guiding of the capsule endoscope 200, and therefore, it is not necessary for the guidance device 20 to detect the position of the capsule endoscope 200 with a high degree of accuracy. For this reason, by decreasing the strength of the capsule detection magnetic field, the electric power that is consumed by the capsule endoscope 200 can be suppressed. Thereafter, the operation of the capsule endoscope 200 returns back to the main routine.

On the other hand, when the distance from the image-capturing target is less than the predetermined value (step S201: No), the detection magnetic field controller 127 increases the power supply provided to the detection magnetic field generation unit 126 (step S203), and the strength of the capsule detection magnetic field is increased. In this case, when the distance from the image-capturing target is short, more specifically, when the capsule endoscope 200 is close to the image-capturing target, the position and the posture of the capsule endoscope 200 is finely adjusted or the capsule endoscope 200 is held in a stationary state, and therefore, it is necessary to improve the guiding operation performance of the capsule endoscope 200 by the guidance device 20. For this reason, by increasing the strength of the detection magnetic field, the guidance device 20 is caused to detect the position of the capsule endoscope 200 with a high degree of accuracy. It should be noted that the power supply provided to the detection magnetic field generation unit 126 is already increased, it is sufficient to only maintain the level of the power supply in step S203, and thereafter, the operation of the capsule endoscope 200 returns back to the main routine.

As described above, according to Embodiment 2-1, in accordance with the environment in which the capsule endoscope 200 is placed, the strength of the detection magnetic field generated by the capsule endoscope 200 is controlled, and therefore, while the electric power that is consumed by the capsule endoscope 200 is suppressed, the guidance device 20 can efficiently perform guiding operation of the capsule endoscope 200.

### (Modification 2-1-1)

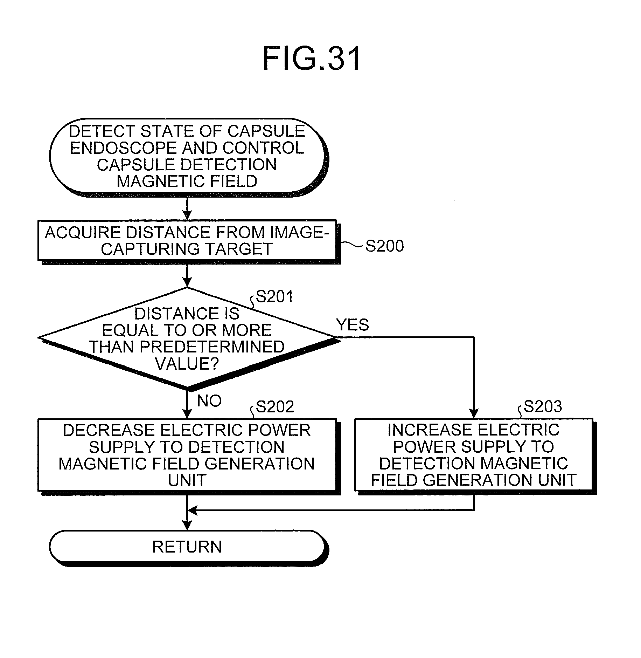

In the control operation of state detection and capsule detection magnetic field of the capsule endoscope (step S14), when the strength of the detection magnetic field is to be changed based on the distance between the image-capturing target and the capsule endoscope 200, control which is opposite to Embodiment 2-1 may be performed. FIG. 31 is a flowchart illustrating operation of the capsule endoscope 200 in step S14 according to Modification 2-1-1.

More specifically, when the distance acquired by the distance acquiring unit 201 is less than a predetermined value (step S201: No), the detection magnetic field controller 127 decreases the power supply provided to the detection magnetic field generation unit 126 (step S202). In this case, when the distance is short, the deviation of the image-capturing range is small in accordance with the position change of the capsule endoscope 200, and therefore, even when the strength of the detection magnetic field is decreased and the accuracy of detection of the capsule endoscope 200 by the guidance device 20 is somewhat reduced, the acquired image is hardly affected. Therefore, the electric power that is consumed by the capsule endoscope 200 can be suppressed by decreasing the power supply provided to the detection magnetic field generation unit 126.

On the other hand when the distance is determined to be equal to or more than the predetermined value (step S201: Yes), the detection magnetic field controller 127 increases the power supply provided to the detection magnetic field generation unit 126 (step S203). This is because, when the distance is far, the deviation of the image-capturing range is large in accordance with the position change of the capsule endoscope 200, and therefore, the accuracy of detection of the capsule endoscope 200 by the guidance device 20 is improved by increasing the strength of the detection magnetic field. It should be noted that when the power supply provided to the detection magnetic field generation unit 126 is already increased, it is sufficient to only maintain the level of the power supply in step S203.

### (Modification 2-1-2)

Subsequently, Modification 2-1-2 of Embodiment 2-1 will be explained.

The distance acquiring unit 201 may acquire the distance from the image-capturing target based on light adjustment information when each image is captured. As described above, when the distance from the image-capturing target is close, light adjustment information for decreasing the amount of light emission is generated, and when the distance from the image-capturing target is far, light adjustment information for increasing the amount of light emission is generated. Accordingly, the distance acquiring unit 201 is provided with a storage unit for storing a table including the distance from the image-capturing target and the quantity of light adjustment of the illumination unit 111 associated with each other. The corresponding distance is extracted from the table based on the light adjustment information acquired from the image-capturing controller 121, so that necessary distance information can be acquired.

### (Modification 2-1-3)

Subsequently, Modification 2-1-3 of Embodiment 2-1 will be explained.

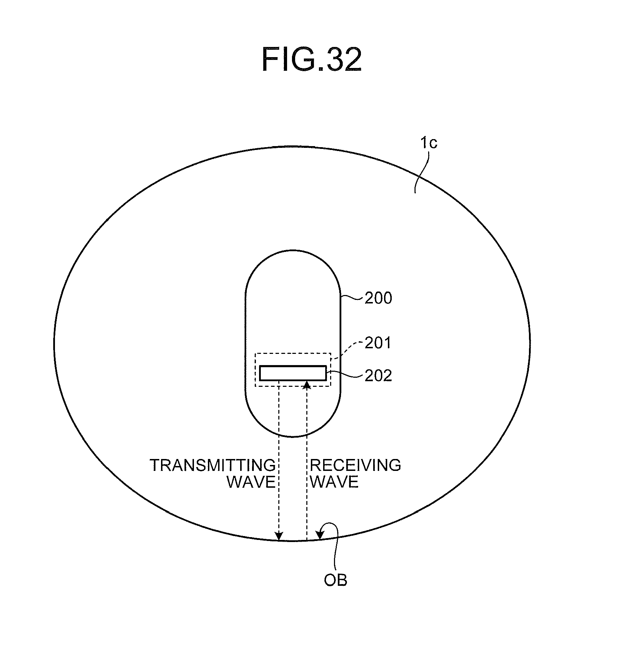

FIG. 32 is a schematic view for explaining the configuration and the operation of a distance acquiring unit 201 according to Modification 2-1-3. The distance acquiring unit 201 may acquire the distance from the image-capturing target, using an ultrasonic wave. In a specific configuration, as illustrated in FIG. 32, the distance acquiring unit 201 is provided with an ultrasonic wave sensor 202 which transmits an ultrasonic wave (transmitting wave) toward an image-capturing target OB in a body cavity 1c of a subject and which receives an ultrasonic wave (receiving wave) reflected by the image-capturing target. In this case, the distance acquiring unit 201 measures a time from when the ultrasonic wave is transmitted to when it is received, and calculates, from this time, the distance from the capsule endoscope 200 to the image-capturing target OB.

### (Modification 2-1-4)

Subsequently, Modification 2-1-4 of Embodiment 2-1 will be explained.

The distance acquiring unit 201 mainly acquires the distance from the image-capturing target using laser. In a specific configuration, the distance acquiring unit 201 is provided with a laser light source for emitting laser light toward the image-capturing target and an optical detection device for detecting laser light reflected by the image-capturing target. In this case, the distance acquiring unit 201 measures the time from when the laser light is emitted to when it is detected, and calculates, from this time, the distance from the capsule endoscope 200 to the image-capturing target.

### (Modification 2-1-5)

Subsequently, Modification 2-1-5 of Embodiment 2-1 will be explained.

The distance acquiring unit 201 may acquire the distance from the image-capturing target based on the color of the image-capturing target. In this case, the farther the image-capturing target is from the capsule endoscope 200, the darker the color of the image-capturing target becomes, and the closer the image-capturing target is to the capsule endoscope 200, the brighter the color of the image-capturing target becomes, and therefore, the distance can be acquired from the brightness of the image-capturing target. Accordingly, in Modification 2-1-5, the distance acquiring unit 201 is provided with a storage unit for storing a table including the brightness and the distance of the image-capturing target associated with each other, and a color sensor made of a three-channel photodiode having sensitivity for each of R, G, B, for example. In this case, the distance acquiring unit 201 calculates the brightness of the image-capturing target from the output value of the color sensor, and extracts the distance from the table based on the brightness, thus capable of acquiring necessary distance information.

### (Embodiment 2-2)

Subsequently, Embodiment 2-2 of the present invention will be explained.

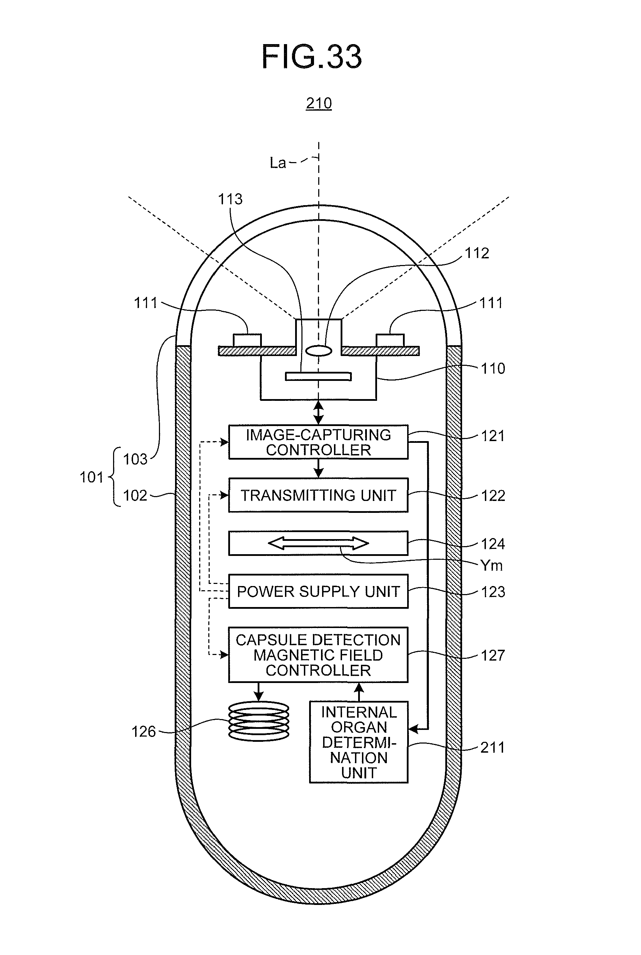

FIG. 33 is a schematic sectional view illustrating an example of an internal structure of a capsule endoscope provided in a medical system according to Embodiment 2-2. It should be noted that the configuration of the entire medical system according to Embodiment 2-2 is the same as what has been illustrated in FIG. 1.

As illustrated in FIG. 33, a capsule endoscope 210 according to Embodiment 2-2 includes an internal organ determination unit 211 instead of the distance acquiring unit 201 as illustrated in FIG. 29. The internal organ determination unit 211 determines the type of the internal organ of the subject where the capsule endoscope 210 is currently passing, as the environment where the capsule endoscope 210 is placed.

More specifically, the internal organ determination unit 211 retrieves the image data from the image-capturing controller 121, and executes image processing for extracting, e.g., color feature data from the in-vivo image corresponding to the image data, thus determining the type of the internal organ. More specifically, when the color feature data of the in-vivo image is, for example, red-like color, the internal organ determination unit 211 determines that the internal organ where the capsule endoscope 210 is passing is the stomach. When the color feature data of the in-vivo image is, for example, yellow-like color, the internal organ determination unit 211 determines that the internal organ where the capsule endoscope 210 is passing is the small intestine. When the color feature data of the in-vivo image is, for example, white-like color, the internal organ determination unit 211 determines that the internal organ where the capsule endoscope 210 is passing is the large intestine.

Subsequently, the operation of the capsule endoscope 210 will be explained. The operation of the entire capsule endoscope 210 is the same as what has been illustrated in FIG. 8, but the contents of the control operation of state detection and capsule detection magnetic field of the capsule endoscope (step S14) are different from those of Embodiment 1-1.

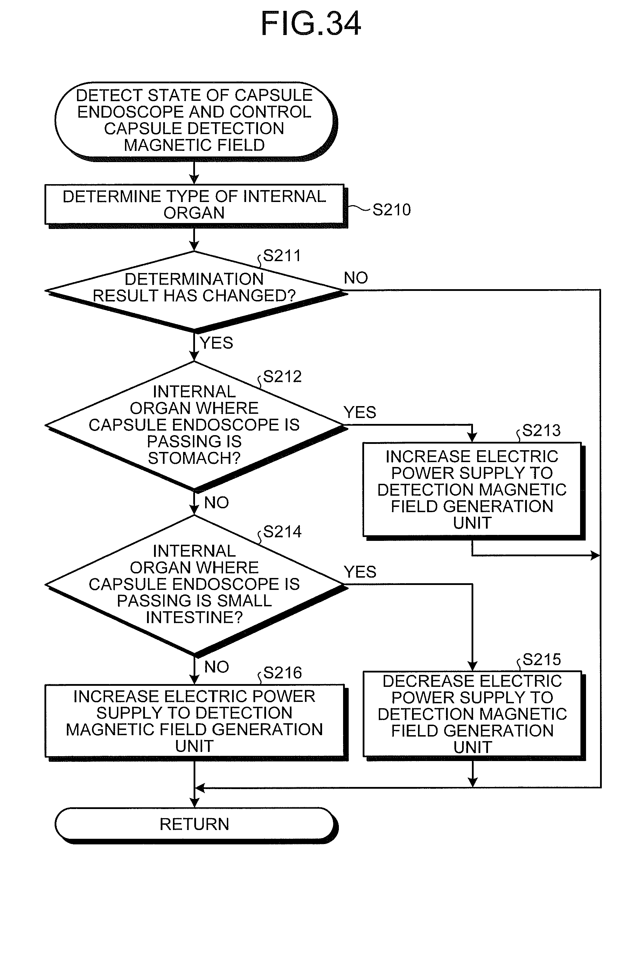

FIG. 34 is a flowchart illustrating operation of the capsule endoscope 210 in step S14.

First, in step S210, the internal organ determination unit 211 applies predetermined image processing on the image data retrieved from the image-capturing controller 121, and determines the internal organ of the subject where the capsule endoscope 210 passes.

In step S211 subsequent thereto, the detection magnetic field controller 127 determines whether the determination result given by the internal organ determination unit 211 has been changed from the previous determination result. More specifically, the detection magnetic field controller 127 determines that the determination result has been changed, e.g., when the capsule endoscope 210 moves from the esophagus to the stomach, or when the capsule endoscope 210 moves from the stomach to the small intestine, or when the capsule endoscope 210 moves from the small intestine to the large intestine. When the determination result is not changed (step S211: No), the operation of the capsule endoscope 210 returns back to the main routine.

When the determination result has been changed (step S211: Yes), and the internal organ where the capsule endoscope 210 passes is determined to be the stomach (step S212: Yes), then the detection magnetic field controller 127 increases the power supply provided to the detection magnetic field generation unit 126 (step S213). In this case, when the capsule endoscope 210 passes a large space such as the stomach, the capsule endoscope 210 can move relatively freely in the horizontal direction and the vertical direction. For this reason, it is necessary for the guidance device 20 to guide the position and the posture of the capsule endoscope 210 in more details, and the position and the posture information therefor is required. Accordingly, with the capsule endoscope 210, the power supply provided to the detection magnetic field generation unit 126 is increased and the strength of the detection magnetic field is increased, so that the accuracy of detection of the capsule endoscope 210 by the guidance device 20 is improved. It should be noted that the power supply provided to the detection magnetic field generation unit 126 is already increased, it is sufficient to only maintain the level of the power supply in step S213.

The internal organ where the capsule endoscope 210 is passing is determined to be the small intestine (step S212: No, step S214: Yes), the detection magnetic field controller 127 decreases the power supply provided to the detection magnetic field generation unit 126 (step S215). In this case, while the capsule endoscope 210 passes a narrow space such as the small intestine, the capsule endoscope 210 moves substantially along the lengthwise direction of the enteric canal. For this reason, it is not necessary for the guidance device 20 to guide the position and the posture of the capsule endoscope 210 in more details, and therefore, it is not necessary to detect the capsule endoscope 210 with a high degree of accuracy. Accordingly, the capsule endoscope 210 decreases the power supply provided to the detection magnetic field generation unit 126, thus alleviating the electric power consumed.

When the internal organ where the capsule endoscope 210 is passing is determined to be the large intestine (step S214: No), the detection magnetic field controller 127 increases the power supply provided to the detection magnetic field generation unit 126 (step S216). The power supply at this occasion is configured to be lower than the value when the capsule endoscope 210 is passing the stomach. In this case, when the capsule endoscope 210 moves from the small intestine to the large intestine, the diameter of the enteric canal is large, and therefore, the range where the capsule endoscope 210 can move also becomes slightly larger. Accordingly, the capsule endoscope 210 slightly increases the power supply provided to the detection magnetic field generation unit 126, and makes the strength of the capsule detection magnetic field be stronger than that in a case of the small intestine, so that the accuracy of detection of the capsule endoscope 210 by the guidance device 20 is improved. Accordingly, while the accuracy of guiding of the capsule endoscope 210 is improved, the electric power that is consumed by the capsule endoscope 210 can be suppressed as compared with the case where the capsule endoscope 210 passes the stomach.

Determination methods for determining the type of the internal organ include not only the calculation of the color feature data but also various kinds of image processing such as pattern matching. For example, a memory storing a reference image for each internal organ is provided, and the reference image and an image captured by the capsule endoscope 210 are compared, and in accordance with the comparison result, the internal organ can be determined. In this case, between the reference image and the captured image, a pattern such as color and shape for each internal organ may be compared. Alternatively, a feature point on a reference image may be determined in advance, and the feature point and a feature point on a captured image are compared, so that the type of the internal organ may be determined. The feature point may be feature of recession/projection, distance between a recession and a projection, an outline of recession/projection, a brightness value of an image, and the like.

### (Modification 2-2-1)

Subsequently, Modification 2-2-1 of Embodiment 2-2 will be explained.

The internal organ determination unit 211 may determine the type of an internal organ from the color of the image-capturing target that is detected in a pinpoint manner. In this case, as described above, in each internal organ, the feature of the color according to the color is observed. More specifically, in a case of the stomach, for example, red-like color becomes intense. In a case of the small intestine, for example, yellow-like color becomes intense. In a case of the large intestine, for example, white-like color becomes intense. Accordingly, the internal organ determination unit 211 is provided with, for example, a color sensor made of a three-channel photodiode having sensitivity for each of R, G, B, for example, and a storage unit for storing a table including the color of the image-capturing target and the type of the internal organ associated with each other. In this case, the internal organ determination unit 211 acquires the color of the image-capturing target (for example, calculating a hue value) from the output value of the color sensor, and refers to the table based on the color, thus capable of detecting the type of the internal organ.

### (Modification 2-2-2)

Subsequently, Modification 2-2-2 of Embodiment 2-2 will be explained.

The type of the internal organ may be determined from the difference in the amount of mucus existing in the internal organ and the property of the mucus (viscosity and pH). Accordingly, the internal organ determination unit 211 may be provided with a mucus sensor for detecting the amount of mucus and the viscosity, and based on the output result of the mucus sensor, the type of the internal organ where the capsule endoscope 210 is passing may be detected. Alternatively, the internal organ determination unit 211 may be provided with a pH sensor, and based on the output value of the pH sensor (pH value), the type of the internal organ where the capsule endoscope 210 is passing may be detected.

Alternatively, a pressure sensor may be provided outside of the capsule endoscope 210, and based on the output value of the pressure sensor, the type of the internal organ may be determined. For example, when, at multiple portions of the capsule endoscope 210, pressure (contact) is detected, the capsule endoscope 210 may be determined to be passing through the small intestine where the diameter of the lumen is small. When no contact pressure is detected for the capsule endoscope 210 or contact pressure is detected in only one direction, the capsule endoscope 210 can determine that the capsule endoscope 210 is passing through the stomach having a large space.

Alternatively, when liquid is introduced into an internal organ (for example, into the stomach) and observation is done while the capsule endoscope 210 is caused to drift about in the liquid, then a water pressure sensor may be provided outside of the capsule endoscope 210. In a large space such as the stomach, liquid can be accumulated. In this case, while the water pressure sensor detects water pressure, the capsule endoscope 210 may be determined to be passing through the stomach. On the contrary, when the water pressure sensor does not detect the water pressure, or when the water pressure sensor detects only local (for example, only at one side) water pressure, then the capsule endoscope 210 is determined to be passing through the internal organ having a narrow space (for example, the small intestine).

### (Modification 2-2-3)

Subsequently, Modification 2-2-3 of Embodiment 2-2 will be explained.

The internal organ where the capsule endoscope 210 is passing may also be determined using, for example, laser light. More specifically, the internal organ determination unit 211 is provided with a set of a laser light source emitting laser light and an optical detection device for detecting laser light reflected by the image-capturing target in two directions in such a manner that the emission directions of the laser light are different from each other (for example, the axial direction and the diameter direction of the capsule endoscope 210). In this case, the internal organ determination unit 211 measures a time from when the laser light is emitted to when it is detected in each direction. Then, the distance from the capsule endoscope 210 to the image-capturing target is calculated in two directions in the space, from the times. The internal organ determination unit 211 finds the spatial distance in accordance with the distances, and determines the internal organ.

### (Modification 2-2-4)

Subsequently, Modification 2-2-4 of Embodiment 2-2 will be explained.

In Embodiment 2-2, the strength of the capsule detection magnetic field is controlled based on the size of the space where the capsule endoscope 210 is passing in accordance with the type of the internal organ (more specifically, the flexibility of the motion of the capsule endoscope 210). However, in accordance with the moving speed of the capsule endoscope 210 in accordance with the type of the internal organ, the strength of the capsule detection magnetic field may be controlled.

For example, in normal circumstances, in the esophagus, the moving speed of the capsule endoscope 210 is increasing, and therefore, the strength of the detection magnetic field is enhanced, and the guidance device 20 is enabled to detect the capsule endoscope 210 with a high degree of accuracy.

The capsule endoscope 210 stays in the stomach for a longer time than in the esophagus, and therefore, when it is confirmed that the capsule endoscope 210 has reached the stomach, the strength of the capsule detection magnetic field may be decreased.

In the small intestine, the moving speed of the capsule endoscope 210 is relatively slow, and therefore, the strength of the capsule detection magnetic field may be decreased.

In the large intestine, the moving speed of the capsule endoscope 210 is faster than the moving speed of the capsule endoscope 210 in the small intestine, and therefore, after it is confirmed that the capsule endoscope 210 moves from the small intestine to the large intestine, it is preferable to increase the strength of the capsule detection magnetic field.

### (Modification 2-2-5)

Subsequently, Modification 2-2-5 of Embodiment 2-2 will be explained.

In Embodiment 2-2, based on the type of the internal organ, the strength of the capsule detection magnetic field is controlled, but characteristic structure such as bleeding and tumor appearing in an image may be detected by image processing, and based on the characteristic structure, the strength of the capsule detection magnetic field may be controlled. For example, when characteristic structure such as bleeding and tumor is detected, it is preferable to increase the power supply provided to the detection magnetic field generation unit 126 and increase the strength of the capsule detection magnetic field, thus improving the accuracy of detection of the capsule endoscope 210 by the guidance device 20.

### (Embodiment 3-1)

Subsequently, Embodiment 3-1 of the present invention will be explained.

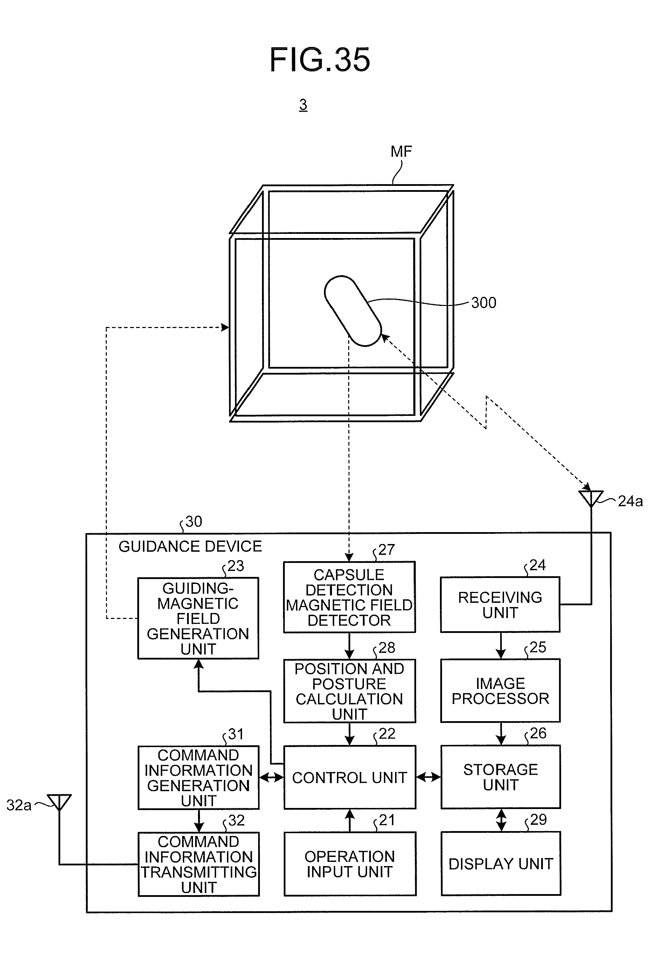

FIG. 35 is a block diagram illustrating a configuration of a medical system according to Embodiment 3-1. As illustrated in FIG. 35, a medical system 3 according to Embodiment 3-1 has a capsule endoscope 300 and a guidance device 30.

In addition to the configuration of the guidance device 20 as illustrated in FIG. 1, the guidance device 30 further includes a command information generation unit 31 and a command information transmitting unit 32. Except the command information generation unit 31 and the command information transmitting unit 32, the configuration and the operation of each unit of the guidance device 30 are the same as those of Embodiment 1-1.

Under the control of the control unit 22, the command information generation unit 31 generates command information for controlling the strength of the capsule detection magnetic field with the capsule endoscope 300 (for example, controlling the power supply provided to the detection magnetic field generation unit 126). In Embodiment 3-1, the command information generation unit 31 generates, as command information, information indicating the strength, the direction, and the gradient of the guiding-magnetic field MF. The command information transmitting unit 32 wirelessly transmits command information generated by the command information generation unit 31 by using a transmitting antenna 32a.

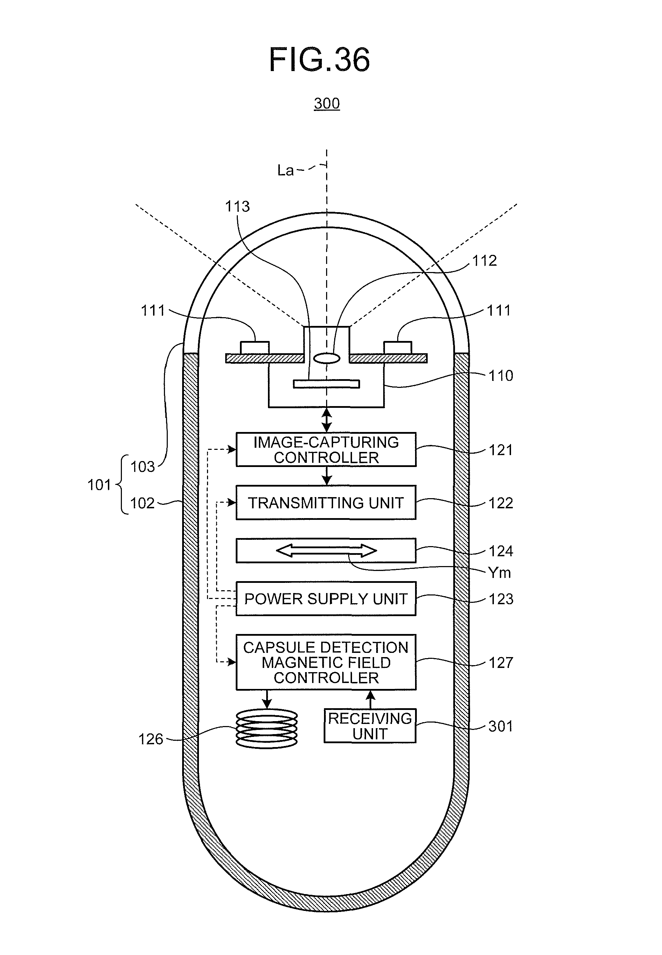

FIG. 36 is a schematic sectional view illustrating an internal configuration of a capsule endoscope 300 as illustrated in FIG. 35. As illustrated in FIG. 3, the capsule endoscope 300 includes a receiving unit 301 for receiving the command information wirelessly transmitted from the guidance device 30, instead of the guiding-magnetic field detector 125 provided in the capsule endoscope 100 as illustrated in FIG. 2. The receiving unit 301 is a form of a state detection unit for detecting the state of the capsule endoscope 300, and detects the guiding-magnetic field MF applied to the capsule endoscope 300 via the received command information. It should be noted that except the receiving unit 301, the configuration and the operation of each unit of the capsule endoscope 300 are the same as those of Embodiment 1 and the modification thereof.

Subsequently, the operation of the capsule endoscope 300 will be explained.

The operation of the entire capsule endoscope 300 is the same as what has been illustrated in FIG. 8, and the contents of the control operation of state detection and capsule detection magnetic field of the capsule endoscope (step S14) are different from those of Embodiment 1-1.

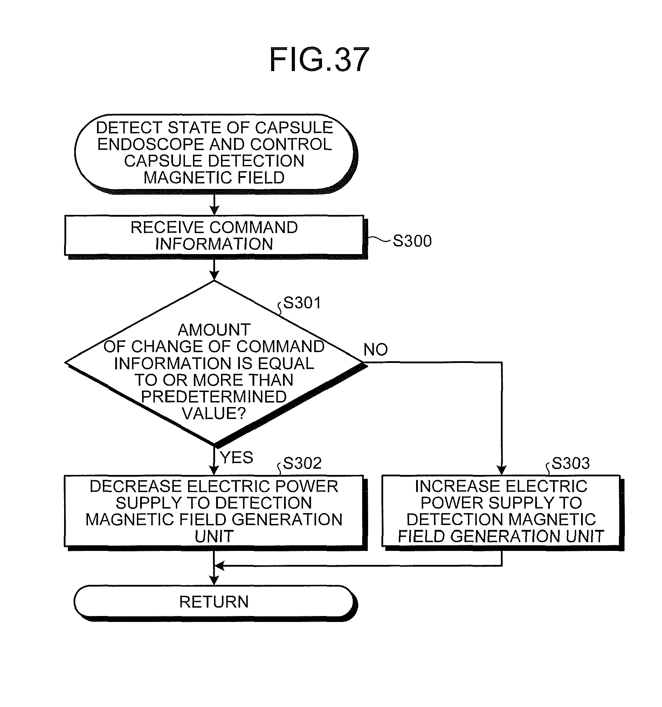

FIG. 37 is a flowchart illustrating operation of the capsule endoscope 300 in step S14.

First, in step S300, the capsule endoscope 300 receives the command information transmitted from the guidance device 30.

In step S301 subsequent thereto, the detection magnetic field controller 127 determines whether the amount of the change of the guiding-magnetic field MF (the amount of the change of the strength and the amount of the change of the direction) is equal to or more than a predetermined threshold value (predetermined value) from the command information given by the receiving unit 301. In this case, the amount of the change of the guiding-magnetic field MF corresponds to the amount of the change of the command information, i.e., the amount of the operation for the operation input unit 21 of the guidance device 30.

When the amount of the change of the guiding-magnetic field MF is equal to or more than a predetermined value (step S301: Yes), the detection magnetic field controller 127 decreases the power supply provided to the detection magnetic field generation unit 126 (step S302). In this case, when the amount of the change of the guiding-magnetic field MF is high, this may be determined that, at the guidance device 30, rough guiding operation is performed on the capsule endoscope 300. In such case, the electric power that is consumed by the capsule endoscope 300 can be suppressed by decreasing the strength of the capsule detection magnetic field. Thereafter, the operation of the capsule endoscope 300 returns back to the main routine.

On the other hand, when the amount of the change of the guiding-magnetic field MF is less than a predetermined value (step S301: No), the detection magnetic field controller 127 increases the power supply provided to the detection magnetic field generation unit 126 (step S303). In this case, when the amount of the change of the guiding-magnetic field MF is low, this may be determined that, at the guidance device 30, detailed guiding operation is performed on the capsule endoscope 300 in order to observe the inside of the subject in details. In such case, the strength of the capsule detection magnetic field is increased, and the accuracy of detection of the capsule endoscope 300 by the guidance device 30 is improved, so that the maneuverability in the guiding operation performed on the capsule endoscope 300 can be improved. It should be noted that when the power supply provided to the detection magnetic field generation unit 126 is already increased, it is sufficient to only maintain the level of the power supply in step S303, and thereafter, the operation of the capsule endoscope 300 returns back to the main routine.

As described above, according to Embodiment 3-1, in accordance with the amount of the operation of the guiding operation by the guidance device 30, the capsule endoscope 300 controls the power supply provided to the detection magnetic field generation unit 126, and accordingly, while the accuracy of detection of the capsule endoscope 300 required for the guiding operation is ensured, the electric power that is consumed by the capsule endoscope 300 can be suppressed.

### (Modification 3-1-1)

The detection magnetic field controller 127 may control the power supply provided to the detection magnetic field generation unit 126 based on information representing the change in the strength of the guiding-magnetic field MF in the command information received from the guidance device 30 (see Embodiment 1-1). Alternatively, the power supply provided to the detection magnetic field generation unit 126 may be controlled based on information representing the change of the gradient magnetic field in the command information received from the guidance device 30 (see Modifications 1-1-2 and 1-1-3).

### (Modification 3-1-2)

The command information generation unit 31 may generate, as command information, information for directly controlling the power supply provided to the detection magnetic field generation unit 126. More specifically, when the guidance device 30 greatly increases the strength of the guiding-magnetic field MF, the command information generation unit 31 generates command information for, e.g., increasing (or maintaining) the strength of the capsule detection magnetic field, or increasing (or maintaining) the power supply provided to the detection magnetic field generation unit 126. On the contrary, when the guidance device 30 decreases the strength of the guiding-magnetic field MF, the command information generation unit 31 generates command information for, e.g., decreasing the strength of the capsule detection magnetic field, or, decreasing the power supply provided to the detection magnetic field generation unit 126. In this case, the detection magnetic field controller 127 may control the power supply provided to the detection magnetic field generation unit 126 simply in accordance with the received command information.

### (Modification 3-1-3)

The command information generation unit 31 may generate, as command information, guiding operation information which is input from the operation input unit 21 (information representing the direction, the posture, and the like for moving the capsule endoscope 300). In this case, when, e.g., the amount of the operation of the guiding operation is high based on the guiding operation information received as the command information, the detection magnetic field controller 127 performs control so as to decrease the power supply provided to the detection magnetic field generation unit 126, and when the amount of the operation of the guiding operation is low, the detection magnetic field controller 127 performs control so as to increase the power supply provided to the detection magnetic field generation unit 126.

Alternatively, the command information generation unit 31 may generate, as command information, information for directly controlling the power supply provided to the detection magnetic field generation unit 126 based on the guiding operation information which is input from the operation input unit 21. For example, when the amount of the operation with the guiding operation in the guidance device 30 is high, the command information generation unit 31 generates command information for decreasing the power supply provided to the detection magnetic field generation unit 126. On the contrary, when the amount of the operation with the guiding operation in the guidance device 30 is low, the command information generation unit 31 generates command information for increasing the power supply provided to the detection magnetic field generation unit 126. In this case, the detection magnetic field controller 127 may control the power supply provided to the detection magnetic field generation unit 126 simply in accordance with the received command information.

### (Embodiment 3-2)

Subsequently, Embodiment 3-2 of the present invention will be explained.

Embodiment 3-2 is characterized in that command information for controlling the strength of the capsule detection magnetic field in the capsule endoscope 300 is generated based on the image data received from the capsule endoscope 300. The configuration of the medical system according to Embodiment 3-2 is the same as what is illustrated in FIG. 35.

In this case, the distance between the capsule endoscope 300 and the image-capturing target can be determined by the brightness of the in-vivo image in which the image-capturing target appears. More specifically, the farther the image-capturing target is away from the capsule endoscope 300, the darker the in-vivo image becomes. The closer the image-capturing target is to the capsule endoscope 300, the brighter the in-vivo image becomes. Accordingly, the control unit 22 causes the image processor 25 to calculate the brightness from the pixel value of a pixel constituting each in-vivo image based on the image data received from the capsule endoscope 300. The command information generation unit 31 generates, as command information, distance information corresponding to the brightness, and causes the command information transmitting unit 32 to transmit it. The distance information can be acquired using calculation expression of distance information stored in the storage unit 26 in advance and a table including the brightness and the distance information associated with each other.

In the capsule endoscope 300, the detection magnetic field controller 127 controls the power supply provided to the detection magnetic field generation unit 126 based on the command information received by the receiving unit 301. More specifically, the distance between the image-capturing target and the capsule endoscope 300 is equal to or more than a predetermined value, the power supply provided to the detection magnetic field generation unit 126 is decreased. On the other hand, when the distance between the image-capturing target and the capsule endoscope 300 is less than the predetermined value, the power supply provided to the detection magnetic field generation unit 126 is increased (see steps S201 to S203 explained in Embodiment 2-1).

Like Modification 2-1-1, it should be noted that when the distance between the image-capturing target and the capsule endoscope 300 is equal to or more than a predetermined value, the capsule endoscope 300 may perform control so as to decrease the power supply provided to the detection magnetic field generation unit 126, and when the distance is less than the predetermined value, the capsule endoscope 300 may perform control so as to increase the power supply provided to the detection magnetic field generation unit 126.

### (Modification 3-2-1)

In the guidance device 30, the command information generation unit 31 may not generate the distance information itself, and may generate, as command information, information for directly controlling the power supply provided to the detection magnetic field generation unit 126 in the capsule endoscope 300 (command information for increasing/decreasing the power supply). For example, when the acquired distance information is equal to or more than a predetermined value, the control unit 22 generates command information for decreasing the power supply provided to the detection magnetic field generation unit 126. On the contrary, when the acquired distance information is less than the predetermined value, the command information generation unit 31 generates command information for increasing the power supply provided to the detection magnetic field generation unit 126. In this case, in the capsule endoscope 300, the detection magnetic field controller 127 may control the power supply provided to the detection magnetic field generation unit 126 simply in accordance with the received command information.

### (Modification 3-2-2)

In the guidance device 30, based on the image data received from the capsule endoscope 300, the internal organ where the capsule endoscope 300 is passing may be determined, and the type of the determined internal organ may be transmitted as command information. Alternatively, in accordance with the type of the determined internal organ, information for controlling the strength of the detection magnetic field with the capsule endoscope 300 may be transmitted as command information.

In an example of determination method of the type of an internal organ, the control unit 22 causes the image processor 25 to execute image processing for extracting color feature data from an in-vivo image corresponding to the image data received from the capsule endoscope 300. Then, when the extracted color feature data is, for example, red-like color, the control unit 22 determines that the internal organ where the capsule endoscope 300 is passing is the stomach. When the color feature data is, for example, yellow-like color, the control unit 22 determines that the internal organ where the capsule endoscope 300 is passing is the small intestine. When the color feature data is, for example, white-like color, the control unit 22 determines that the internal organ where the capsule endoscope 300 is passing is the large intestine.

When the internal organ where the capsule endoscope 300 is passing is determined to be the esophagus, the command information generation unit 31 generates command information for, e.g., increasing the power supply provided to the detection magnetic field generation unit 126. When the capsule endoscope 300 is determined to have reached from the esophagus to the stomach, the command information generation unit 31 generates command information for, e.g., decreasing the power supply provided to the detection magnetic field generation unit 126. When the capsule endoscope 300 is determined to have moved from the stomach to the small intestine, the command information generation unit 31 generates command information for, e.g., keeping on decreasing the power supply provided to the detection magnetic field generation unit 126. When the capsule endoscope 300 is determined to have moved from the small intestine to the large intestine, the command information generation unit 31 generates command information for, e.g., increasing the power supply provided to the detection magnetic field generation unit 126. It should be noted that the reason why such control is performed is the same as what has been explained in Embodiment 2-2.

It should be noted that determination methods for determining the type of the internal organ based on the in-vivo image include not only the method using the color feature data but also various kinds of methods such as pattern matching. For example, the storage unit 26 may store a reference image for each internal organ in advance, and the image processor 25 may compare the reference image and an image captured by the capsule endoscope 300, and in accordance with the comparison result, the internal organ can be determined. In this case, between the reference image and the captured image, a pattern such as color and shape for each internal organ may be compared. Alternatively, a feature point on a reference image may be determined in advance, and the feature point and a feature point on a captured image are compared, so that the type of the internal organ may be determined. The feature point may be feature of recession/projection, distance between a recession and a projection, an outline of recession/projection, a brightness value of an image, and the like.

### (Modification 3-2-3)

The guidance device 30 may extract characteristic structure from an in-vivo image corresponding to image data received from the capsule endoscope 300, and generate command information for controlling the power supply to the detection magnetic field generation unit 126 based on the characteristic structure. For example, when characteristic structure such as bleeding and tumor is detected by image processing of the image processor 25, the command information generation unit 31 generates command information for increasing the power supply provided to the detection magnetic field generation unit 126, and causes the command information transmitting unit 32 to transmit it.

### (Embodiment 3-3)

Subsequently, Embodiment 3-3 of the present invention will be explained.

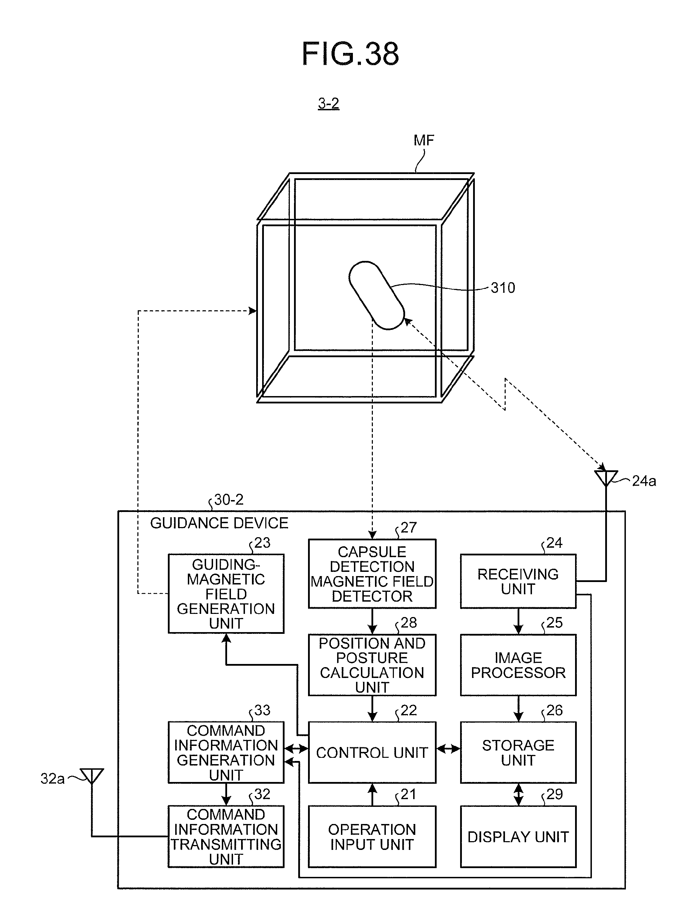

FIG. 38 is a block diagram illustrating a configuration of a medical system according to Embodiment 3-3. As illustrated in FIG. 38, a medical system 3-2 according to Embodiment 3-3 includes a capsule endoscope 310 and a guidance device 30-2.

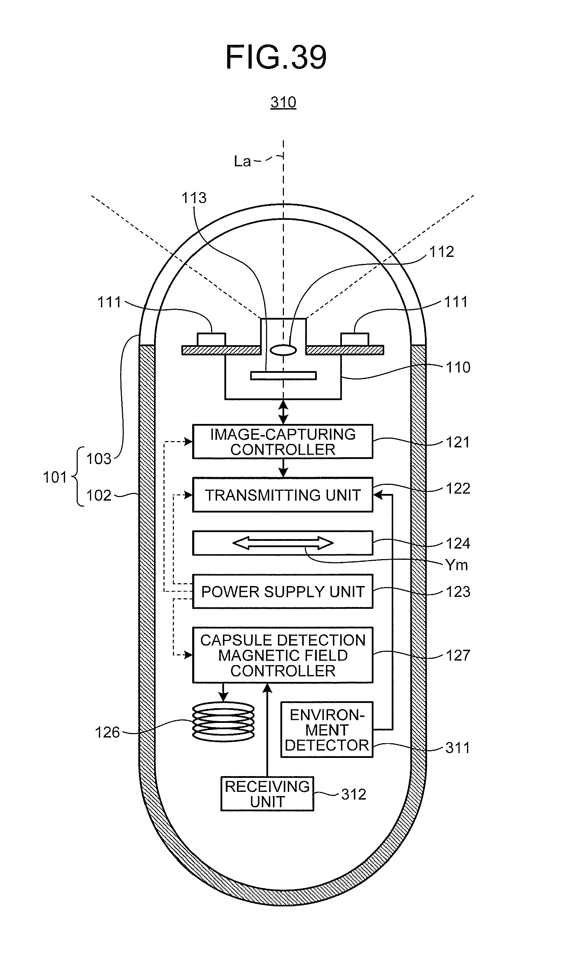

FIG. 39 is a schematic sectional view illustrating an example of an internal structure of the capsule endoscope 310. As illustrated in FIG. 39, the capsule endoscope 310 is based on the capsule endoscope 100 as illustrated in FIG. 2, but further includes an environment detector 311 for detecting the environment of the capsule endoscope 310 within the subject and causes the transmitting unit 122 to wirelessly transmit it and a receiving unit 312 for receiving information wirelessly transmitted from the guidance device 30-2.

The environment detector 311 detects, for example, the distance between the image-capturing target within the subject and the capsule endoscope 310 as the environment of the capsule endoscope 310. In this case, as a specific configuration of the environment detector 311, for example, an ultrasonic wave sensor may be provided to transmit an ultrasonic wave (transmitting wave) to the image-capturing target OB within the body cavity 1c of the subject, and receive an ultrasonic wave (receiving wave) reflected by the image-capturing target. As the environment detector 311, a laser light source for emitting laser light to the image-capturing target and an optical detection device for detecting laser light reflected by the image-capturing target may be provided. Alternatively, as the environment detector 311, for example, a color sensor (see Modification 2-1-5) made of a three-channel photodiode having sensitivity for each of R, G, B, may be provided. The information detected by the environment detector 311 is wirelessly transmitted as the environment information to the guidance device 30-2.

The receiving unit 312 is also a state detection unit for receiving the information transmitted from the guidance device 30-2 and detecting the state in which the capsule endoscope 310 is placed based on the information.

On the other hand, in contrast to the guidance device 30 as illustrated in FIG. 35, the guidance device 30-2 includes a command information generation unit 33 instead of the command information generation unit 31. The command information generation unit 33 operates under the control of the control unit 22, and acquires the environment information that is transmitted from the capsule endoscope 310 and received by the receiving unit 24, and generates command information for controlling the capsule detection magnetic field with the capsule endoscope 310.

More specifically, when the environment detector 311 is an ultrasonic wave sensor, the command information generation unit 33 calculates the distance between the image-capturing target and the capsule endoscope 310 from a transmission time and a reception time of the ultrasonic wave received as the environment information. When the environment detector 311 is a laser light source and an optical detection device, the command information generation unit 33 calculates the distance from emission timing and detection timing of the laser light. Further, when the environment detector 311 is a color sensor, the command information generation unit 33 calculates the distance between the image-capturing target and the capsule endoscope 310 based on information including the brightness and the distance of the image-capturing target which are associated with each other and the detection result of the color sensor. In this case, the storage unit 26 of the guidance device 30-2 stores a table including the brightness and the distance of the image-capturing target associated with each other in advance.

Then, the command information generation unit 33 generates information for controlling the power supply provided to the detection magnetic field generation unit 126 with the capsule endoscope 310 based on the calculated distance. More specifically, when the distance between the image-capturing target and the capsule endoscope 310 is equal to or more than a predetermined value, the command information generation unit 33 generates command information for decreasing the power supply provided to the detection magnetic field generation unit 126, and when the distance is less than the predetermined value, the command information generation unit 33 generates command information for increasing the power supply provided to the detection magnetic field generation unit 126. Alternatively, when the distance between the image-capturing target and the capsule endoscope 310 is equal to or more than a predetermined value, the command information generation unit 33 may generate command information for decreasing the power supply provided to the detection magnetic field generation unit 126, and when the distance is less than the predetermined value, the command information generation unit 33 may generate command information for increasing the power supply provided to the detection magnetic field generation unit 126.

The command information transmitting unit 32 wirelessly transits the command information generated by the command information generation unit 33 to the capsule endoscope 310. This command information is received by the receiving unit 312 of the capsule endoscope 310. The detection magnetic field controller 127 controls the power supply for the detection magnetic field generation unit 126 in accordance with the command information.

### (Modification 3-3-1)

The command information generated by the command information generation unit 33 may be the calculated distance itself based on the environment information received by the capsule endoscope 310. In this case, the detection magnetic field controller 127 performs the control based on, e.g., the increase and the decrease of the power supply provided to the detection magnetic field generation unit 126 based on the received command information (distance information).

### (Modification 3-3-2)

Subsequently, Modification 3-3-2 will be explained.

The guidance device 30-2 may determine the internal organ where the capsule endoscope 310 is passing, based on the environment information detected and transmitted by the environment detector 311, and generate and transmit command information for controlling the power supply provided to the detection magnetic field generation unit 126 in accordance with the type of the internal organ where the capsule endoscope 310 is passing.

For example, as the environment detector 311 of the capsule endoscope 310, a mucus sensor and a pH sensor for detecting mucus in the internal organ may be provided, and the output results of the mucus sensor or the pH sensor is transmitted as the environment information to the guidance device 30-2. In this case, in the guidance device 30-2, the command information generation unit 33 determines the type of the internal organ where the capsule endoscope 310 is passing based on the received environment information, generates the following command information in accordance with the type of the internal organ, and causes the command information transmitting unit 32 to transmit it.

More specifically, when the internal organ where the capsule endoscope 310 is passing is determined to be the esophagus, the command information generation unit 33 generates command information for, e.g., increasing the power supply provided to the detection magnetic field generation unit 126. When the capsule endoscope 310 is determined to have reached from the esophagus to the stomach, the command information generation unit 33 generates command information for, e.g., decreasing the power supply provided to the detection magnetic field generation unit 126. When the capsule endoscope 310 is determined to have moved from the stomach to the small intestine, the command information generation unit 33 generates command information for, e.g., keeping on decreasing the power supply provided to the detection magnetic field generation unit 126. When the capsule endoscope 310 is determined to have moved from the small intestine to the large intestine, the command information generation unit 33 generates command information for, e.g., increasing the power supply provided to the detection magnetic field generation unit 126. It should be noted that the reason why such control is performed is the same as what has been explained in Embodiment 2-2.

In the capsule endoscope 310, the detection magnetic field controller 127 controls the power supply to the detection magnetic field generation unit 126 in accordance with the command information received by the receiving unit 312.

### (Modification 3-3-3)

The command information which the guidance device 30-2 transmits to the capsule endoscope 310 may be information itself that represents the type of the internal organ determined by the command information generation unit 33. In this case, the detection magnetic field controller 127 makes the same determination based on the information representing the type of the internal organ received, and controls the power supply provided to the detection magnetic field generation unit 126.

### (Modification 3-3-4)

The internal organ where the capsule endoscope 310 is passing may be determined not only by the method explained in Modification 3-3-2 but also by various methods.

For example, like Modification 2-2-1, when the capsule endoscope 310 is provided with a color sensor, the storage unit 26 stores a table storing the color of the image-capturing target and the type of the internal organ associated with each other. In this case, the command information generation unit 33 can determine the type of the internal organ based on the table and the color of the image-capturing target acquired from the output value of the color sensor received as the environment information.

When a pressure sensor is provided outside of the capsule endoscope 310 like Modification 2-2-2, the command information generation unit 33 can determine the type of the internal organ based on the output value of the pressure sensor received as the environment information. Alternatively, when liquid is introduced into an internal organ (for example, into the stomach) and observation is done while the capsule endoscope 310 is caused to drift about in the liquid, a water pressure sensor is provided outside of the capsule endoscope 310. In this case, the command information generation unit 33 can determine the type of the internal organ in accordance with the output value of the water pressure sensor received as the environment information.

When a laser light source and an optical detection device are provided on the capsule endoscope 310 like Modification 2-2-3, the command information generation unit 33 can determine the type of the internal organ based on the distance in the space in two directions based on the time from when the laser light is emitted to when it is detected which is received as the environment information.

### (Modification 4)

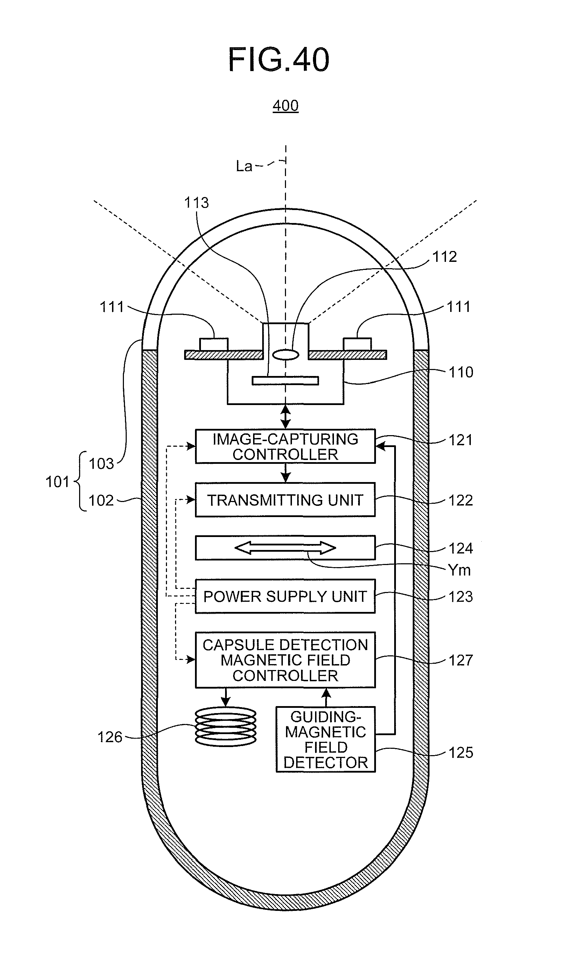

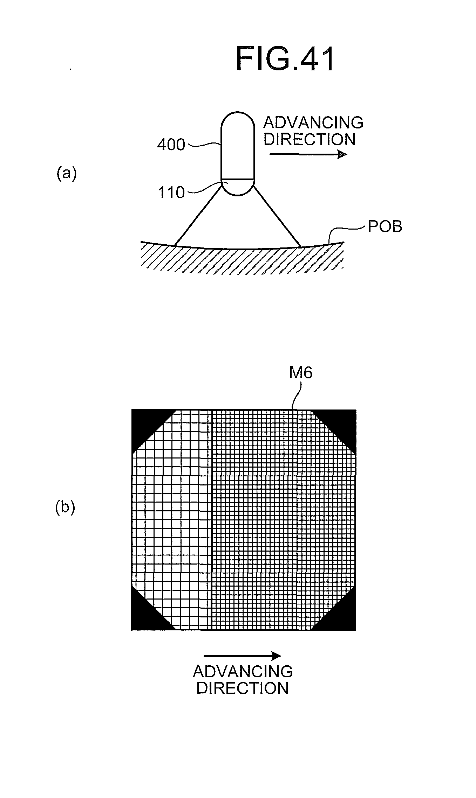

In Embodiments 1-1 to 3-3 and Modification thereof explained above, the electric power consumed by the capsule endoscope is suppressed by controlling the strength of the capsule detection magnetic field, but the electric power consumed by the capsule endoscope may be saved by controlling parameters other than the above. For example, like a capsule endoscope 400 as illustrated in FIG. 40, the output result of the guiding-magnetic field detector 125 may be input into the image-capturing controller 121, and in accordance with the output result, the amount of data wirelessly transmitted may be reduced by decreasing the image-capturing frame rate during the image-capturing process or increasing the compression rate of image data. For example, as illustrated in FIG. 41(a), when the capsule endoscope 400 advances along an inner wall surface POB of an internal organ in such orientation that the image-capturing unit 110 faces the inner wall surface POB of the internal organ, the amount of data may be decreased as follows. As illustrated in FIG. 41(b), within one in-vivo image M6, detailed information may be displayed by decreasing the compression rate in a region in an advancing direction, and increasing the compression rate in a region behind the advancing direction, so that the amount of data is decreased.

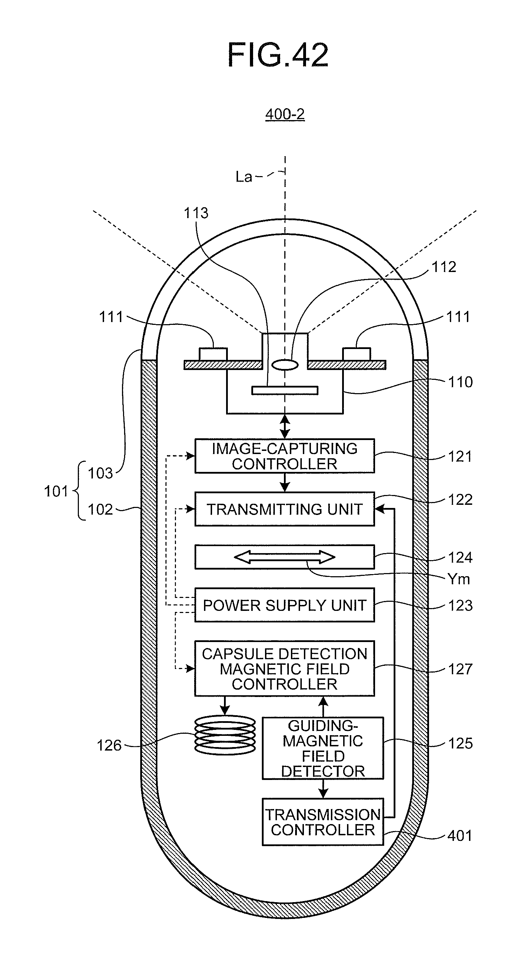

Alternatively, like a capsule endoscope 400-2 as illustrated in FIG. 42, a transmission controller 401 for controlling operation of the transmitting unit 122 may be further provided, and in accordance with the output result of the guiding-magnetic field detector 125, the transmission rate of the image data transmitted by the transmitting unit 122 may be reduced.

In a case where, instead of the guiding-magnetic field detector 125, the capsule endoscope is provided with the motion detector 131 (see FIG. 17), the gradient field detector 141 (see FIG. 23), the distance acquiring unit 201 (see FIG. 29), the internal organ determination unit 211 (see FIG. 33), the environment detector 311 (see FIG. 39), and the like, the image-capturing frame rate, the compression rate of the image, the transmission rate, and the like may be controlled based on the output result of each of these units in the same manner.

### (Modification 5)

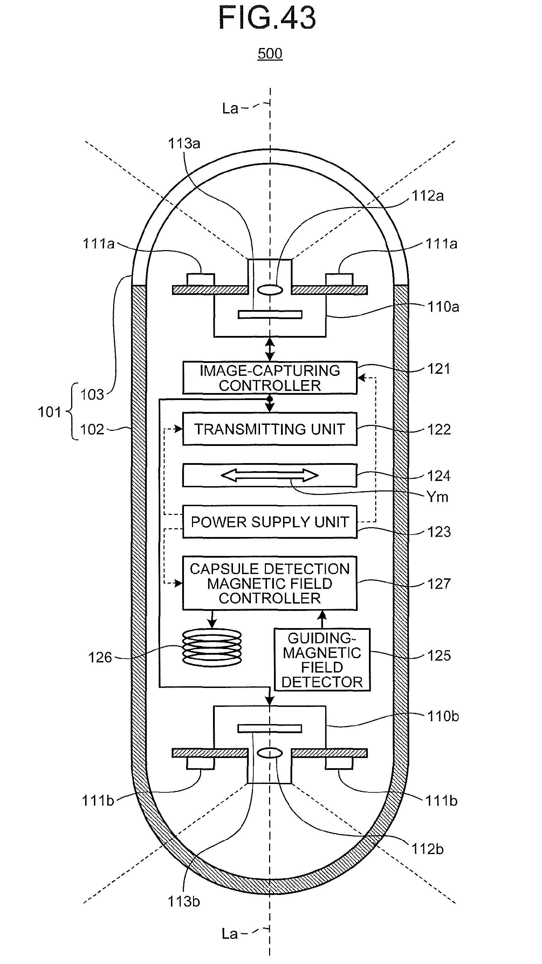

In Embodiments 1-1 to 3-3 and the modifications thereof explained above, a single image-capturing unit capsule has been used, in which an image-capturing unit is provided at an end of the capsule endoscope in the longitudinal axis La. Alternatively, for example, as illustrated in FIG. 43, a multi-image-capturing unit capsule may be used, in which image-capturing units 110a, 110b are respectively provided at both ends of a capsule endoscope 500 in the longitudinal axis La. Like Embodiment 1-1, the image-capturing units 110a, 110b include illumination units 111a, 111b, optical systems 112a, 112b, and image-capturing devices 113a, 113b.

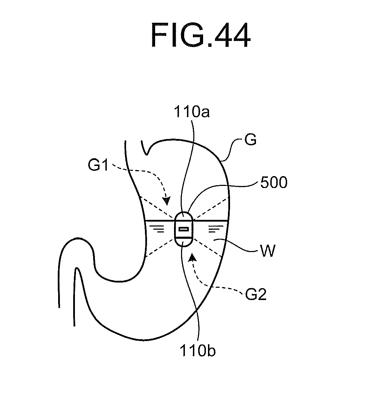

In this case, the capsule endoscope 500 can acquire, at a time, both of the images in the forward and the backward directions with respect to the advancing direction. As illustrated in FIG. 44, when observation is done while the capsule endoscope 500 is caused to drift about in the liquid W in an internal organ (for example, the stomach) into which liquid W is introduced, then both of the images in a vision field range G1 above the capsule endoscope 500 and a vision field range G2 below the capsule endoscope 500 can be acquired at a time.

Even when the capsule endoscope 500 of such multi-image-capturing unit is used, the power supply provided to the detection magnetic field generation unit 126 may be controlled in accordance with the output result of guiding-magnetic field detector 125, or the motion detector 131 (see FIG. 17), the gradient field detector 141 (see FIG. 23), the distance acquiring unit 201 (see FIG. 29), the internal organ determination unit 211 (see FIG. 33), the environment detector 311 (see FIG. 39), and the like provided as the state detection unit instead of the guiding-magnetic field detector 125, like Embodiments 1-1 to 3-3 and the modification thereof.

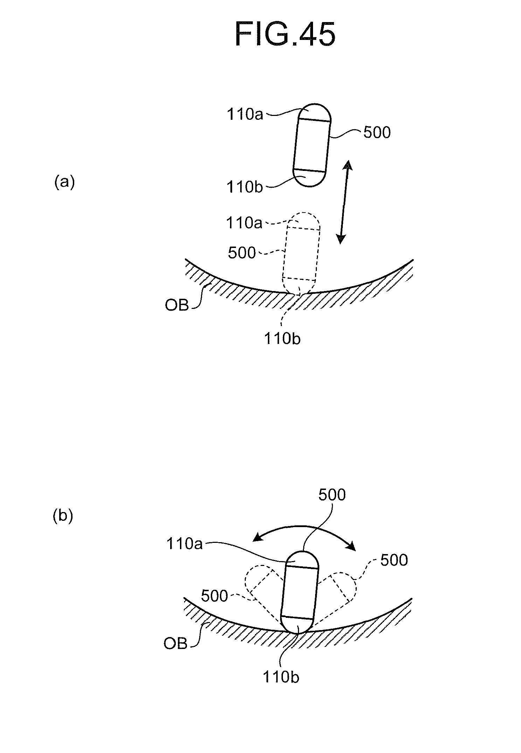

Alternatively, in accordance with the motion of the capsule endoscope 500 (the change of the position and the posture) and the guiding operation for the capsule endoscope 500, the image-capturing frame rate of the image-capturing units 110a, 110b and the compression rate of the image data may be controlled. For example, as illustrated in FIG. 45(a), when the capsule endoscope 500 moves closer to and moves away from the image-capturing target OB, the image-capturing frame rate of the image-capturing unit 110b facing the image-capturing target OB is controlled so that when the capsule endoscope 500 moves closer to image-capturing target OB, the image-capturing frame rate is increased, and when the capsule endoscope 500 moves away from image-capturing target OB, the image-capturing frame rate is decreased, As illustrated in FIG. 45(b), when the capsule endoscope 500 is caused to perform so-called swinging operation, the image-capturing frame rate at the image-capturing unit 110a of which motion is high may be increased, and the image-capturing frame rate at the image-capturing unit 110b of which motion is low may be decreased. In such case, a motion detector and the like respectively corresponding to the image-capturing units 110a, 110b may be provided on the capsule endoscope 500.

When such control is performed, the electric power of the entire capsule endoscope 500 may be suppressed while acquiring necessary image information.

The above-described embodiments and their modifications are just examples for carrying out the present invention, and the present invention is not limited to them. Moreover, various inventions can be derived from combinations of a plurality of components disclosed in the embodiments and their modifications of the present invention. Various modifications can be made according to specifications, and it is obvious from the above disclosures that other various embodiments may be made within the scope of the present invention.

### (Supplementary Note 1)

A capsule medical device which is introduced into a subject and used therein, the capsule medical device including:
a power supply unit;
a capsule detection magnetic field generation unit configured to generate a capsule detection magnetic field, with which a position and/or a posture of the capsule medical device is detected outside the subject, upon receiving electric power provided by the power supply unit;
a state detection unit configured to detect a state of the capsule medical device;
a control unit configured to control power supply from the power supply unit to the capsule detection magnetic field generation unit based on a detection result of the state detection unit; and
a magnetic field response unit made of a magnetic member and configured to respond to a guiding-magnetic field which is a magnetic field applied from outside to the capsule medical device for guiding at least one of the position and the posture of the capsule medical device,
wherein the state detection unit includes a guiding-magnetic field detector configured to detect at least one of a strength, a direction, and a gradient of the guiding-magnetic field, and
the control unit controls the power supply in accordance with a detection result of the guiding-magnetic field detector.

(Supplementary Note 2)

A capsule medical device which is introduced into a subject and is used therein, the capsule medical device including:
a power supply unit;
a capsule detection magnetic field generation unit configured to generate a capsule detection magnetic field, with which a position and/or a posture of the capsule medical device is detected outside the subject, upon receiving electric power provided by the power supply unit;
a state detection unit configured to detect a state of the capsule medical device; and
a control unit configured to control power supply from the power supply unit to the capsule detection magnetic field generation unit based on a detection result of the state detection unit,
wherein when electric power is intermittently provided from the power supply unit to the capsule detection magnetic field generation unit, the control unit controls a supply interval of the electric power based on a detection result of the state detection unit.

### (Supplementary Note 3)

A capsule medical device which is introduced into a subject and is used therein, the capsule medical device including:
a power supply unit;
a capsule detection magnetic field generation unit configured to generate a capsule detection magnetic field, with which a position and/or a posture of the capsule medical device is detected outside the subject, upon receiving electric power provided by the power supply unit;
a state detection unit configured to detect a state of the capsule medical device; and
a control unit configured to control power supply from the power supply unit to the capsule detection magnetic field generation unit based on a detection result of the state detection unit,
wherein the capsule detection magnetic field generation unit includes a plurality of coils of which impedances are different from each other, and
the control unit selects a coil for providing electric power from among the plurality of coils based on a detection result of the state detection unit.

### (Supplementary Note 4)

A medical system including:
a capsule medical device which is introduced into a subject and is used therein, the capsule medical device including
a power supply unit,
a capsule detection magnetic field generation unit configured to generate a capsule detection magnetic field, with which a position and/or a posture of the capsule medical device is detected outside the subject, upon receiving electric power provided by the power supply unit,
a state detection unit configured to detect a state of the capsule medical device,and
a control unit configured to control power supply from the power supply unit to the capsule detection magnetic field generation unit based on a detection result of the state detection unit; and
an external device provided outside of the subject, the external device including
a capsule detection magnetic field detector configured to detect the magnetic field generated by the capsule detection magnetic field generation unit, and
a position and posture detection unit configured to detect at least one of the position and the posture of the capsule medical device based on the detection result of the capsule detection magnetic field detector.

### (Supplementary Note 5)

The medical system according to Supplementary Note 4, wherein the external device further includes a command information transmitting unit configured to transmit command information for controlling the power supply provided by the power supply unit,
the state detection unit receives the command information transmitted from the command information transmitting unit, and
the control unit controls the power supply based on the command information received by the state detection unit.

### (Supplementary Note 6)

The medical system according to Supplementary Note 5, wherein the capsule medical device further includes a magnetic field response unit made of a magnetic member and configured to respond to a guiding-magnetic field which is a magnetic field applied from outside to the capsule medical device for guiding at least one of the position and the posture of the capsule medical device,
the external device further includes a guiding-magnetic field generation unit configured to generate the guiding-magnetic field, and
the command information is information relating to the guiding-magnetic field generated by the external device.

### (Supplementary Note 7)

The medical system according to Supplementary Note 5, wherein the capsule medical device further includes:
an image-capturing unit configured to acquire image information by capturing an image inside of the subject; and
a transmitting unit configured to wirelessly transmit the image information and/or related information related to the image information, and
wherein the external device further includes
a receiving unit configured to receive the image information and/or related information related to the image information transmitted by the transmitting unit; and
a command information generation unit configured to generate, as the command information, information about a distance between the capsule medical device and the subject, based on at least one of the image information and the related information received by the receiving unit.

### (Supplementary Note 8)

The medical system according to Supplementary Note 5, wherein the capsule medical device further includes:
an environment detection unit configured to generate environment information by detecting environment representing characteristic of a space around itself; and
a transmitting unit configured to wirelessly transmit the environment information,
the external device further includes:
   a receiving unit configured to receive the environment information transmitted by the transmitting unit; and
   a command information generation unit configured to generate, as the command information, information about a distance between the capsule medical device and the subject based on the environment information received by the receiving unit.

### (Supplementary Note 9)

The medical system according to Supplementary Note 5, wherein the capsule medical device further includes:
an environment detection unit configured to generate environment information by detecting environment representing characteristic of a space around itself; and
a transmitting unit configured to wirelessly transmit the environment information,
the external device further includes:
   a receiving unit configured to receive the environment information transmitted by the transmitting unit; and
   a command information generation unit configured to generate, as the command information, information about the type of the internal organ of the subject where the capsule medical device is passing, based on the environment information received by the receiving unit.

### Reference Signs List

- 1, 1-2, 3, 3-2: MEDICAL SYSTEM
- 1a: SUBJECT
- 1b: BED
- 1c: BODY CAVITY
- 20, 20-2: GUIDANCE DEVICE
- 20a: GRADIENT FIELD GENERATION UNIT
- 21: OPERATION INPUT UNIT
- 22: CONTROL UNIT
- 23: GUIDING-MAGNETIC FIELD GENERATION UNIT
- 24: RECEIVING UNIT
- 24a: ANTENNA
- 25: IMAGE PROCESSOR
- 26: STORAGE UNIT
- 27: CAPSULE DETECTION MAGNETIC FIELD DETECTOR
- 28: POSITION AND POSTURE CALCULATION UNIT
- 28a: FILTER
- 28b: AMPLIFICATION DEVICE
- 28c: A/D CONVERTER (A/D)
- 28d: FFT CALCULATOR (FFT)
- 28e: OSITION AND POSTURE INFORMATION CALCULATION UNIT
- 29: DISPLAY UNIT
- 31, 33: COMMAND INFORMATION GENERATION UNIT
- 32: COMMAND INFORMATION TRANSMITTING UNIT
- 32a: TRANSMITTING ANTENNA
- 41, 42: JOY STICK
- 44U: UP BUTTON
- 44B: DOWN BUTTON
- 45: CAPTURE BUTTON
- 46: APPROACH BUTTON
- 100, 130, 130-2, 140, 150, 200, 210, 300, 310, 400, 500: CAPSULE ENDOSCOPE
- 101: CAPSULE-SHAPED CASING
- 102: CYLINDRICAL CASING
- 103: DOME-SHAPED CASING
- 110, 110a, 110b: IMAGE-CAPTURING UNIT
- 111: ILLUMINATION UNIT
- 112: OPTICAL SYSTEM
- 113: IMAGE-CAPTURING DEVICE
- 121: IMAGE-CAPTURING CONTROLLER
- 122: TRANSMITTING UNIT
- 123: POWER SUPPLY UNIT
- 124: PERMANENT MAGNET
- 125, 151: GUIDING-MAGNETIC FIELD DETECTOR
- 126: CAPSULE DETECTION MAGNETIC FIELD GENERATION UNIT
- 127: CAPSULE DETECTION MAGNETIC FIELD CONTROLLER
- 131, 152: MOTION DETECTOR
- 141: GRADIENT FIELD DETECTOR
- 201: DISTANCE ACQUIRING UNIT
- 202: ULTRASONIC WAVE SENSOR
- 211: INTERNAL ORGAN DETERMINATION UNIT
- 301: RECEIVING UNIT
- 311: ENVIRONMENT DETECTOR
- 401: TRANSMISSION CONTROLLER

## Claims

1. A capsule medical device which is introduced into a subject and used therein, the capsule medical device comprising:
a power supply unit;
a capsule detection magnetic field generation unit configured to generate a capsule detection magnetic field, with which a position and/or a posture of the capsule medical device is detected outside the subject, upon receiving electric power provided by the power supply unit;
a state detection unit configured to detect a state of the capsule medical device; and
a control unit configured to control power supply from the power supply unit to the capsule detection magnetic field generation unit based on a detection result of the state detection unit.

2. The capsule medical device according to claim 1, further comprising:
a magnetic field response unit made of a magnetic member and configured to respond to a guiding-magnetic field which is a magnetic field applied from outside to the capsule medical device for guiding at least one of the position and the posture of the capsule medical device,
wherein the state detection unit includes a guiding-magnetic field detector configured to detect at least one of a strength, a direction, and a gradient of the guiding-magnetic field, and
the control unit controls the power supply in accordance with a detection result of the guiding-magnetic field detector.

3. The capsule medical device according to claim 2, wherein the guiding-magnetic field detector detects the strength of the guiding-magnetic field, and
the control unit controls the power supply provided to the capsule detection magnetic field generation unit in accordance with the strength.

4. The capsule medical device according to claim 2, wherein the guiding-magnetic field detector detects change in the strength of the guiding-magnetic field, and
the control unit controls the power supply provided to the capsule detection magnetic field generation unit in accordance with an amount of the change of the strength.

5. The capsule medical device according to claim 2, wherein the guiding-magnetic field detector detects gradient of the guiding-magnetic field, and
the control unit controls the power supply provided to the capsule detection magnetic field generation unit in accordance with the gradient.

6. The capsule medical device according to claim 2, wherein the guiding-magnetic field detector detects a direction of the guiding-magnetic field, and
the control unit controls the power supply provided to the capsule detection magnetic field generation unit in accordance with a magnitude of an angle formed by the direction and a magnetization direction of the magnetic field response unit.

7. The capsule medical device according to claim 1, wherein the state detection unit includes a motion detector configured to detect motion of the capsule medical device, and
The control unit controls the power supply based on the motion of the capsule medical device detected by the motion detector.

8. The capsule medical device according to claim 7, wherein the motion detector is configured to detect a physical quantity corresponding to acceleration of the capsule medical device, and
the control unit is configured to reduce turns off the power supply provided to the capsule detection magnetic field generation unit when the acceleration determined from a determination result of the motion detector is either equal to or more than a predetermined value or equal to or less than the predetermined value.

9. The capsule medical device according to claim 1, further comprising a magnetic field response unit made of a magnetic member and configured to respond to a guiding-magnetic field which is a magnetic field applied from outside to the capsule medical device for guiding the capsule medical device,
wherein the state detection unit includes:
a guiding-magnetic field detector configured to detect the guiding-magnetic field;
a motion detector configured to detect motion of the capsule medical device; and
a comparison determination unit configured to compare a guiding-magnetic field detection result of the guiding-magnetic field detector and a motion detection result of the motion detector,
wherein the control unit controls the power supply based on a result acquired by comparing the guiding-magnetic field detection result of the guiding-magnetic field detector and the motion detection result of the motion detector.

10. The capsule medical device according to claim 9, wherein the control unit decreases the power supply provided to the capsule detection magnetic field generation unit when the direction of the gradient of the guiding-magnetic field and the moving direction of the capsule medical device are determined to be the same.

11. The capsule medical device according to claim 9, wherein the control unit decreases the power supply provided to the capsule detection magnetic field generation unit when the direction of the guiding-magnetic field and the posture of the capsule medical device are determined to be the same.

12. The capsule medical device according to claim 1, wherein
the state detection unit includes a distance acquiring unit configured to acquire a distance between the capsule medical device and the subject, and
the control unit controls the power supply based on the distance acquired by the distance acquiring unit.

13. The capsule medical device according to claim 13, wherein the control unit decreases the power supply provided to the capsule detection magnetic field generation unit when the distance is either equal to or more than a predetermined value or equal to or less than the predetermined value.

14. The capsule medical device according to claim 1, wherein
the state detection unit includes an internal organ determination unit configured to determine a type of an internal organ of the subject where the capsule medical device passes, and
the control unit controls the power supply based on the type of an internal organ determined by the internal organ determination unit.

15. A medical system comprising:
a capsule medical device according to claim 1; and
an external device provided outside of the subject, the external device including
a capsule detection magnetic field detector configured to detect the magnetic field generated by the capsule detection magnetic field generation unit,
a position and posture detection unit configured to detect at least one of the position and the posture of the capsule medical device based on the detection result of the capsule detection magnetic field detector, and
a command information transmitting unit configured to transmit command information for controlling the power supply provided by the power supply unit,
wherein the state detection unit receives the command information transmitted from command information transmitting unit, and
the control unit controls the power supply based on the command information received by the state detection unit.
